# EUROPEAN PATENT SPECIFICATION

(11) **EP 2 671 587 B1**
(45) Date of publication and mention of the grant of the patent: **26.07.2017**
(21) Application number: 12742051.1
(22) Date of filing: 01.02.2012
(51) Int. Cl.: A61K 31/496, A61K 9/70, A61K 47/04, A61K 47/10, A61K 47/12, A61K 47/14, A61K 47/22, A61K 47/32, A61K 47/44, A61P 25/18, A61P 43/00

(54) **PATCH PREPARATION**
PFLASTERPRÄPARAT
PRÉPARATION DE TIMBRE TRANSDERMIQUE

(30) Priority: 02.02.2011 JP 2011021185; 02.02.2011 JP 2011021192
(43) Date of publication of application: 11.12.2013
(73) Proprietor: NITTO DENKO CORPORATION, Osaka 567-8680 (JP)
(72) Inventor: OKADA, Yasuaki, Ibaraki-shi Osaka 567-8680 (JP); OKADA, Katsuhiro, Ibaraki-shi Osaka 567-8680 (JP); NISHIMURA, Masato, Ibaraki-shi Osaka 567-8680 (JP); KAWAHARADA, Yuji, Ibaraki-shi Osaka 567-8680 (JP); MAEDA, Hiroo, Ibaraki-shi Osaka 567-0878 (JP); YAMAMOTO, Kazumitsu, Ibaraki-shi Osaka 567-0878 (JP); TANAKA, Masayasu, Ibaraki-shi Osaka 567-0878 (JP)
(74) Representative: Grünecker Patent- und Rechtsanwälte PartG mbB
(86) International application number: PCT/JP2012/052310
(87) International publication number: WO 2012/105622

(56) References cited:
- EP-A1- 2 036 559
- EP-A1- 2 671 593
- WO-A1-02/32431
- WO-A1-2005/102393
- WO-A1-2007/142295
- JP-A- 3 106 813
- JP-A- 9 208 463
- JP-A- H07 506 562
- JP-A- H10 504 552
- JP-A- 2000 044 904
- JP-A- 2003 526 678
- JP-A- 2005 179 312
- JP-A- 2008 208 084
- US-A- 4 737 360

## Description

### Technical Field

The present invention relates to a patch preparation to be used by adhering to a skin surface or mucosal surface.

### Background Art

2-(4-Ethyl-1-piperazinyl)-4-(4-fluorophenyl)-5,6,7,8,9,10-hexahydrocycloocta[b]pyridine (generic name "blonanserin"; hereinafter sometimes to be referred to as "compound A") is a serotonin-dopamine antagonist (SDA), and disclosed in patent document 1. Blonanserin shows higher affinity for dopamine D2 and serotonin 5-HT2 receptor as compared to existing antipsychotic, haloperidol, and is useful as an antipsychotic agent.

As transdermal absorption preparations of antipsychotic, those of perospirone and the like are already known and, as for blonanserin, transdermal administration is indicated as one of the administration routes therefor (patent document 2).

A transdermal administration of a drug can avoid first pass effect of the liver since the drug can be directly absorbed from the capillary of the skin surface or mucosal surface. In transdermal administration, moreover, since a drug is released in a sustained manner, side effects caused by absorption of a large amount of the drug in a short time can be reduced. Therefore, transdermal administration is one of the effective means of drug administration.

On the other hand, since the skin functions as a barrier to prevent invasion of a foreign substance from the outside, it is difficult to deliver a drug into the body in an amount necessary and sufficient for providing efficacy by simply applying or adhering the drug to the skin.

To deliver a drug into the body in an amount necessary and sufficient for providing efficacy, the concentration of a drug in a patch preparation may be increased. Generally, however, a high drug concentration may increase irritation to the skin. Therefore, the permeation amount of a drug is sometimes increased by adding, to a preparation, a substance aiming at increasing the skin permeation of the drug.

As disclosed in patent document 2, a patch preparation containing blonanserin (hereinafter to be also referred to as "blonanserin-containing patch preparation") itself is known. As a substance having an action to promote skin permeation of blonanserin, lactic acid, crotamiton and the like are known as described in patent document 2. However, many of the substances having a transdermal absorption-promoting effect show high skin irritation. Therefore, even when the concentration of a drug in a preparation is reduced, the skin irritation sometimes becomes high by the use of a substance having a skin permeability-promoting action.

Moreover, since a patch preparation has a greater contact area with the air, the stability is an important problem.

In the meantime, in an attempt to mitigate skin irritation by an adhesive, a patch preparation having an adhesive layer added with a liquid component compatible with the adhesive is conventionally known. However, a patch preparation containing an adhesive added with such liquid component easily causes problems such as transfer of a liquid component to an adherend (skin and mucous membrane to be an adhesion target) or a support, and the like, depending on the object of use. Thus, for example, patent document 3 describes, as an adhesive for dealing with such problems, an adhesive layer wherein 40 - 80 wt% of the adhesive is insolubilized by crosslinking, and teaches that said adhesive layer shows improved cohesive force while having an appropriate adhesive force. However, even such an adhesive layer may be associated with a problem of decreased adhesiveness of the adhesive layer in the presence of a sweat component due to sweating. Patent document 4 describes an adhesive which avoids the aforementioned problem by adding metal chloride to the adhesive layer and prevents a decrease in the cohesive force of the adhesive layer even in the presence of a sweat component due to sweating. However, it does not consider imparting a sufficient adhesive force to an adhesive layer, and the adhesive layer has a room for further improvement. On the other hand, while patent document 2 describes a preparation of 2-(4-ethyl-1-piperazinyl)-4-(4-fluorophenyl)-5,6,7,8,9,10-hexahydrocycloocta[b]pyridine (generic name "blonanserin") containing various permeation enhancers such as lactic acid, it does not at all consider adhesiveness in the presence of water and the like.

### [Document List]

### [patent documents]

patent document 1: JP-B-H7-47574
patent document 2: WO2007/142295
patent document 3: JP-A-H6-319793
patent document 4: JP-A-2007-16019

### SUMMARY OF THE INVENTION

### Problems to be Solved by the Invention

In view of the above-mentioned situation, a first problem to be solved by the present invention is provision of a blonanserin patch preparation capable of maintaining a blood concentration sufficient for exerting the efficacy of blonanserin and sufficiently reducing skin irritation, and showing good preservation stability.

A second problem to be solved by the present invention is provision of a patch preparation sufficiently enhanced in not only the cohesive force of an adhesive layer and skin permeability of blonanserin, but also adhesiveness in the presence of water.

### Means of Solving the Problems

The present inventors have conducted intensive studies in an attempt to solve the aforementioned first problem and found that the maintenance of the blood concentration of blonanserin and reduction of the skin irritation by lactic acid can be simultaneously achieved by adding sesame oil to an adhesive layer containing blonanserin and lactic acid, and the preservation stability of the preparation can also be improved by a particular additive. Based on such findings, they have conducted further studies and completed the present invention.

In addition, the present inventors have conducted intensive studies in an attempt to solve the aforementioned second problem and found that the above-mentioned problem can be solved by adding a particular filler to an adhesive layer containing blonanserin and lactic acid, that is, the adhesiveness in the presence of water can be sufficiently enhanced, which resulted in the completion of the present invention.

Accordingly, the present specification comprises two patch preparations, and the first aspect of the present invention is as follows.
[1] A patch preparation comprising a support and an adhesive layer formed on one surface of the support, wherein the adhesive layer comprises
   (i) 2-(4-ethyl-1-piperazinyl)-4-(4-fluorophenyl)-5,6,7,8,9,10-hexahydrocycloocta[b]pyridine or a physiologically acceptable acid addition salt thereof,
   (ii) an acrylic polymer,
   (iii) lactic acid,
   (iv) sesame oil and
   (v-1) one or more kinds of additives selected from the group consisting of 2-mercaptobenzimidazole, 2,6-di-tert-butyl-4-methylphenol and propyl gallate, and wherein the adhesive layer further comprises (vi) an organic liquid component which is a fatty acid ester formed of a fatty acid having a carbon number of 8 to 18 and a monohydric alcohol having a carbon number of 1 to 18. (hereinafter to be also referred to as "the patch preparation (I) of the present invention").
[2] The patch preparation of the above-mentioned [1], wherein the content of the lactic acid in 100 wt% of the adhesive layer is 0.1 - 10 wt%.
[3] The patch preparation of the above-mentioned [1] or [2], wherein the content of the sesame oil in 100 wt% of the adhesive layer is 3 - 20 wt%.
[4] The patch preparation of any of the above-mentioned [1] to [3], wherein the content of the additive in 100 wt% of the adhesive layer is 0.3 - 10 wt%.
[5] The patch preparation of any of the above-mentioned [1] to [4], wherein the adhesive layer further comprises (vi) an organic liquid component.
[6] The patch preparation of the above-mentioned [5], wherein the organic liquid component comprises isopropyl myristate (hereinafter sometimes to be referred to as "IPM").
[7] The patch preparation of the above-mentioned [5] or [6], wherein the total content of the organic liquid component and sesame oil in the adhesive layer is 5 - 60 wt%.
[8] The patch preparation of any of the above-mentioned [1] to [7], wherein the adhesive layer is subjected to a crosslinking treatment.
[9] The patch preparation of any of the above-mentioned [1] to [8], wherein the acrylic polymer comprises at least one kind selected from the group consisting of a 2-ethylhexylacrylate/acrylic acid/N-vinyl-2-pyrrolidone copolymer, a 2-ethylhexylacrylate/2-hydroxyethylacrylate/vinyl acetate copolymer and a 2-ethylhexylacrylate/acrylic acid copolymer.
[10] The patch preparation of any of the above-mentioned [1] to [8], wherein the acrylic polymer comprises a 2-ethylhexylacrylate/acrylic acid/N-vinyl-2-pyrrolidone copolymer.
[11] The patch preparation of any of the above-mentioned [1] to [10], wherein the lactic acid includes DL-lactic acid and L-lactic acid.
   The second aspect of the present invention is as follows.
[12] A patch preparation comprising a support and an adhesive layer formed on one surface of the support, wherein the adhesive layer comprises
   (i) 2- (4-ethyl-1-piperazinyl) -4- (4-fluorophenyl) - 5,6,7,8,9,10-hexahydrocycloocta[b]pyridine or a physiologically acceptable acid addition salt thereof,
   (ii) an acrylic polymer,
   (iii) lactic acid and
   (v-2) a filler comprising one or more kinds selected from the group consisting of magnesium aluminometasilicate, polyvinylpyrrolidone and an aminoalkyl methacrylate copolymer, and wherein the adhesive layer further comprises (vi) an organic liquid component which is a fatty acid ester formed of a fatty acid having a carbon number of 8 to 18 and a monohydric alcohol having a carbon number of 1 to 18. (hereinafter to be also referred to as "the patch preparation (II) of the present invention").
[13] The patch preparation of the above-mentioned [12], wherein the filler comprises magnesium aluminometasilicate.
[14] The patch preparation of the above-mentioned [13], wherein the magnesium aluminometasilicate is an amorphous composite oxide wherein aluminum, magnesium and a silicon atom are three-dimensionally polymerized via an oxygen atom.
[15] The patch preparation of any one of the above-mentioned [12] to [14], wherein the adhesive layer is crosslinked.
[16] The patch preparation of any one of the above-mentioned [12] to [15], wherein the content of the lactic acid in the adhesive layer is 0.1 - 10 wt% in 100 wt% of the adhesive layer.
[17] The patch preparation of any one of the above-mentioned [12] to [16], wherein the content of the filler in the adhesive layer is 0.1 - 40 wt% in 100 wt% of the adhesive layer.
[18] The patch preparation of any one of the above-mentioned [12] to [17], wherein the adhesive layer further comprises (vi) an organic liquid component.
[19] The patch preparation of the above-mentioned [18], wherein the organic liquid component comprises isopropyl myristate.
[20] The patch preparation of any one of the above-mentioned [12] to [19], wherein the adhesive layer comprises (iv) sesame oil.
[21] The patch preparation of any one of the above-mentioned [12] to [20], wherein the adhesive layer further comprises one or more kinds of additives selected from (v-1) 2-mercaptobenzimidazole, 2,6-di-tert-butyl-4-methylphenol and propyl gallate.
[22] The patch preparation of the above-mentioned [21], wherein the content of the additive in the adhesive layer is 0.3 - 10 wt% in 100 wt% of the adhesive layer.
[23] The patch preparation of any one of the above-mentioned [12] to [22], wherein the acrylic polymer comprises at least one kind selected from the group consisting of a 2-ethylhexylacrylate/acrylic acid/N-vinyl-2-pyrrolidone copolymer, a 2-ethylhexylacrylate/2-hydroxyethylacrylate/vinyl acetate copolymer and a 2-ethylhexylacrylate/acrylic acid copolymer.
[24] The patch preparation of any one of the above-mentioned [12] to [23], wherein the acrylic polymer comprises a 2-ethylhexylacrylate/acrylic acid/N-vinyl-2-pyrrolidone copolymer.
[25] The patch preparation of any one of the above-mentioned [12] to [24], wherein the lactic acid is DL-lactic acid or L-lactic acid.
[26] A method for transdermally administering 2-(4-ethyl-1-piperazinyl)-4-(4-fluorophenyl)-5,6,7,8,9,10-hexahydrocycloocta[b]pyridine, comprising applying the patch preparation of any one of the above-mentioned [1] to [25] to a skin of a patient.
[27] A method for treating schizophrenia, comprising applying the patch preparation of any one of the above-mentioned [1] to [25] to a skin of a patient with schizophrenia. Effect of the Invention

The patch preparation (I) of the present invention (the first aspect of the present invention) can provide a blonanserin patch preparation which is superior in the stability of blonanserin during preservation, can maintain a blood concentration sufficient to exert efficacy of blonanserin when in use, and sufficiently reduces skin irritation.

On the other hand, the patch preparation (II) of the present invention (the second aspect of the present invention) can suppress a decrease in the adhesiveness of the patch preparation containing lactic acid in the presence of water, while maintaining a permeation promoting effect afforded by the lactic acid, and can suppress detachment from the skin and the like, by adding at least one kind of filler selected from the group consisting of magnesium aluminometasilicate, polyvinylpyrrolidone and an aminoalkyl methacrylate copolymer to the adhesive layer. Therefore, the second aspect of the present invention can provide a patch preparation superior in both the skin permeability of blonanserin or a physiologically acceptable acid addition salt thereof, and adhesiveness in the presence of water.

### Description of Embodiments

The first and the second aspects of the present invention are each explained in the following according to preferable embodiments thereof.

### 1. The first aspect of the present invention

(i) 2- (4-Ethyl-1-piperazinyl) -4- (4-fluorophenyl) -5, 6, 7, 8, 9, 10-hexahydrocycloocta[b]pyridine (generic name "blonanserin") relating to the first aspect of the present invention is a compound represented by the following formula: 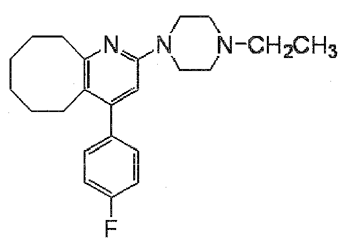 , which is a serotonin•dopamine•antagonist (or dopamine• serotonin•antagonist) as mentioned above, and is commercially available as an antipsychotic agent.

Compound A may be a free base or a physiologically acceptable acid addition salt thereof. Examples of acid addition salts with organic acid include, but are not limited to, formate, acetate, lactate, adipate, citrate, tartrate, methanesulfonate, fumarate, maleate and the like, and examples of acid addition salts with inorganic acid include hydrochloride, sulfate, nitrate, phosphate and the like. Furthermore, compound A or a physiologically acceptable acid addition salt thereof may be any of solvate, hydrate and non-hydrate. Any one kind may be used alone or two or more kinds thereof may be used in combination.

The above-mentioned compound A or a physiologically acceptable acid addition salt thereof can be produced by, for example, the method described in JP-B-H7-47574 (patent document 1) or a method analogous thereto. The produced compound A or a physiologically acceptable acid addition salt thereof may be pulverized as appropriate by a generally-used method.

While the content of the "compound A or a physiologically acceptable acid addition salt thereof " to be contained in the patch preparation (I) of the present invention needs to be determined according to the age, symptom and the like of the patient who receives the administration, and is not particularly limited, it is generally about 0.1 - about 50 wt%, preferably, though depending on the area of the patch preparation, about 0.1 - about 40 wt%, more preferably about 0.1 - about 30 wt%, further preferably about 0.5 - about 50 wt%, still more preferably about 0.5 - about 40 wt%, yet further preferably about 0.5 - about 30 wt%, in terms of compound A, in 100 wt% of the adhesive layer. Here, "in terms of compound A" means that, when compound A is in the form of a physiologically acceptable acid addition salt or compound A contains crystal water, the amount corresponding to the acid added to the acid addition salt or the crystal water is not included in the weight of compound A.

The adhesive layer in the first aspect of the present invention is formed at least on one surface of a support, and the adhesive layer contains at least, in addition to (i) compound A or a physiologically acceptable acid addition salt thereof, (ii) an acrylic polymer, (iii) lactic acid, (iv) sesame oil and (v-1) an additive, which are described below.

The patch preparation (I) of the present invention contains a support and an adhesive layer formed on at least one surface of the support. Where necessary, it may have a release sheet on the surface opposite from the support side of the adhesive layer. In addition, the form of the patch preparation may be a sheet with any shape or a wound form like a roll.

While the above-mentioned support is not particularly limited, it is preferably a material that does not permit compound A or a physiologically acceptable acid addition salt thereof, lactic acid, sesame oil and the like in the adhesive layer to pass through the support and be lost from the back face thereof (face of the support on the side opposite from the side of the adhesive layer) to decrease the content thereof in the adhesive layer, that is, a material having impermeability to compound A or a physiologically acceptable acid addition salt thereof, lactic acid and sesame oil. As mentioned below, in an embodiment wherein the adhesive layer contains an organic liquid component, a material impermeable to compound A or a physiologically acceptable acid addition salt thereof, lactic acid, sesame oil and organic liquid component is preferable.

Specific examples of the above-mentioned support satisfying the impermeability include a single film of polyester (e.g., polyethylene terephthalate (PET) etc.), nylon, polyvinyl chloride, polyethylene, polypropylene, ethylene-vinyl acetate copolymer, polytetrafluoroethylene, ionomer resin and the like, a metal foil, and a laminate film wherein two or more kinds of films selected therefrom are laminated and the like. Of these, to improve adhesiveness (anchor property) of a support to an adhesive layer, it is preferable to use, as a support, a laminate film of a non-porous film made from the above-mentioned material and the following porous film, and form the adhesive layer on the porous film side. The thickness of the non-porous film is preferably 2 - 100 µm, more preferably 2 - 50 µm.

The porous film is not particularly limited as long as the anchor property to an adhesive layer is improved and, for example, paper, woven fabric, non-woven fabric (e.g., polyester (e.g., polyethylene terephthalate (PET) and the like) non-woven fabric and the like), a single film of the above-mentioned film (e.g., polyester, nylon, Saran (trade name), polyethylene, polypropylene, ethylene-vinyl acetate copolymer, polyvinyl chloride, ethylene-ethyl acrylate copolymer, polytetrafluoroethylene, metal foil, polyethylene terephthalate and the like, and a laminate film wherein two or more kinds of films selected therefrom are laminated and the like), which is mechanically perforated, and the like can be mentioned. Particularly, paper, woven fabric and non-woven fabric (e.g., polyester non-woven fabric, polyethylene terephthalate non-woven fabric and the like) are preferable from the aspects of flexibility of the support. For example, when the porous film is woven fabric or non-woven fabric, the fabric weight is preferably 5 - 30 g/m² to improve anchor property.

A laminate film for the support is manufactured by a known manufacture method of laminate films such as dry lamination method, wet lamination method, extrusion lamination method, hot melt lamination method, coextrusion lamination method and the like.

While the thickness of the support is not particularly limited, it is preferably 2 - 200 µm, more preferably 10 - 50 µm. When it is less than 2 µm, the handling property such as self-supporting property tends to decrease, and when it exceeds 200 µm, an uncomfortable feeling (a feeling of stiffness) occurs, and the followability tends to decrease.

(ii) The first aspect of the present invention contains an acrylic polymer as an adhesive in the adhesive layer. Compound A or a physiologically acceptable acid addition salt thereof shows high solubility in an acrylic polymer as compared to rubber polymers, and a small possibility of forming a crystal of compound A or a physiologically acceptable acid addition salt thereof in the adhesive layer. Therefore, an acrylic polymer is advantageous, and the adhesive is preferably constituted by an acrylic polymer alone. When an adhesive polymer other than an acrylic polymer is contained as the adhesive, the adhesive polymer other than an acrylic polymer is contained in not more than 10 wt% relative to the total weight of the adhesive in the adhesive layer (acrylic polymer is 90 wt% or more). Examples of the adhesive polymer other than an acrylic polymer include rubber adhesive polymers, silicone adhesive polymers and the like.

As the acrylic polymer in the first aspect of the present invention, an acrylic polymer comprising a unit of alkyl(meth)acrylate as a main constitution unit is preferable. The acrylic polymer comprising alkyl(meth)acrylate as a main constitution unit is preferably a copolymer of alkyl(meth)acrylate (the first monomer component) and a vinyl monomer having a functional group capable of being involved in a crosslinking reaction (the second monomer component), or a copolymer wherein a monomer other than these (the third monomer component) is further copolymerized, from the aspects of adhesiveness to human skin, solubility of drug during formulation of a preparation and the like.

Examples of the above-mentioned alkyl(meth)acrylate (the first monomer component) include alkyl(meth)acrylate wherein the alkyl group is a linear, branched chain or cyclic alkyl group having a carbon number of 1 to 18 (e.g., methyl, ethyl, n-propyl, isopropyl, n-butyl, isobutyl, tert-butyl, sec-butyl, n-pentyl, isopentyl, neopentyl, n-hexyl, isohexyl, cyclohexyl, 3-methylpentyl, n-heptyl, cycloheptyl, n-octyl, 2-ethylhexyl, cyclooctyl, n-nonyl, cyclononyl, n-decyl, cyclodecyl, n-undecyl, n-dodecyl, n-tridecyl and the like) and the like, preferably alkyl(meth)acrylate wherein the alkyl group is a linear, branched chain or cyclic alkyl group having a carbon number of 4 to 18 (e.g., n-butyl, isobutyl, tert-butyl, sec-butyl, n-pentyl, isopentyl, neopentyl, n-hexyl, isohexyl, cyclohexyl, 3-methylpentyl, n-heptyl, cycloheptyl, n-octyl, 2-ethylhexyl, cyclooctyl, n-nonyl, cyclononyl, n-decyl, cyclodecyl, n-undecyl, n-dodecyl, n-tridecyl and the like). To particularly confer adhesiveness at ambient temperature, use of a monomer component that decreases the glass transition temperature of the polymer is preferable. Thus, alkyl(meth)acrylate wherein the alkyl group is a linear, branched chain or cyclic alkyl group having a carbon number of 4 to 8 (e.g., n-butyl, isobutyl, tert-butyl, sec-butyl, n-pentyl, isopentyl, neopentyl, n-hexyl, isohexyl, cyclohexyl, 3-methylpentyl, n-heptyl, cycloheptyl, n-octyl, 2-ethylhexyl, cyclooctyl and the like) is more preferable, and alkyl(meth)acrylate wherein the alkyl group is n-butyl, 2-ethylhexyl or cyclohexyl is particularly preferable.

Specific, particularly preferable examples of the alkyl(meth)acrylate (the first monomer component) include butyl acrylate, 2-ethylhexylacrylate, 2-ethylhexylmethacrylate, cyclohexylacrylate and cyclohexylmethacrylate. Of these, 2-ethylhexylacrylate is most preferable. One or more kinds of these alkyl(meth)acrylates (the first monomer component) can be used in combination.

In the above-mentioned vinyl monomer having a functional group capable of being involved in a crosslinking reaction (the second monomer component), examples of the functional group capable of being involved in a crosslinking reaction include a hydroxy group, a carboxy group, a vinyl group and the like, preferably a hydroxy group and a carboxy group. Specific examples of said monomer (the second monomer component) include hydroxyethyl(meth)acrylate, hydroxypropyl(meth)acrylate, (meth)acrylic acid, itaconic acid, maleic acid, maleic anhydride, methaconic acid, citraconic acid, glutaconic acid and the like. Of these, acrylic acid, methacrylic acid and hydroxyethylacrylate are preferable, and acrylic acid is most preferable, since they are easily available. One or more kinds of these monomers (the second monomer component) can be used in combination.

In addition, the above-mentioned other monomer (the third monomer component) is mainly used for adjusting the cohesive force of the adhesive layer, adjusting solubility and releasability of a drug (compound A or a physiologically acceptable acid addition salt thereof) and the like. Examples of the monomer (the third monomer component) include vinyl esters such as vinyl acetate, vinyl propionate and the like; vinyl ethers such as methyl vinyl ether, ethyl vinyl ether and the like; vinyl amides such as N-vinyl-2-pyrrolidone, N-vinylcaprolactam and the like; alkoxy(meth)acrylates such as methoxyethyl(meth)acrylate, ethoxyethyl(meth)acrylate, tetrahydrofurfuryl(meth)acrylate and the like; hydroxy group-containing monomers such as hydroxypropyl(meth)acrylate, α-hydroxymethyl acrylate and the like (such hydroxy group-containing monomer is used as the third monomer component and is not involved in a crosslinking reaction); (meth)acrylic acid derivatives having an amide group such as (meth)acrylamide, dimethyl(meth)acrylamide, N-butyl(meth)acrylamide, N-methylol(meth)acrylamide and the like; aminoalkyl(meth)acrylates such as aminoethyl(meth)acrylate, dimethylaminoethyl(meth)acrylate, tert-butylaminoethyl(meth)acrylate and the like; alkoxyalkyleneglycol(meth)acrylates such as methoxyethyleneglycol(meth)acrylate, methoxydiethyleneglycol(meth)acrylate, methoxypolyethylene glycol(meth)acrylate, methoxypolypropyleneglycol(meth)acrylate and the like; (meth)acrylonitrile; sulfo group-containing monomers such as styrene sulfonic acid, ally sulfonic acid, sulfopropyl(meth)acrylate, (meth)acryloyloxynaphthalenesulfonic acid, acrylamidemethylsulfonic acid and the like; vinyl group-containing monomers such as vinyl piperidone, vinyl pyrimidine, vinyl piperazine, vinyl pyrrole, vinyl imidazole, vinyl oxazole, vinyl morpholine etc., and the like. Among these, vinyl esters and vinyl amides are preferable, vinyl ester is preferably vinyl acetate, and vinyl amide is preferably N-vinyl-2-pyrrolidone. One or more kinds of these monomers (the third monomer component) can be used in combination.

When the acrylic polymer is a copolymer of alkyl(meth)acrylate (the first monomer component) and a vinyl monomer having a functional group capable of being involved in a crosslinking reaction (the second monomer component), the copolymerization ratio (first monomer component/second monomer component) is preferably 85 - 99 wt%/1 - 15 wt%, more preferably 90 - 99 wt%/1 - 10 wt%.

When the acrylic polymer is a copolymer of alkyl(meth)acrylate (the first monomer component), a vinyl monomer having a functional group capable of being involved in a crosslinking reaction (the second monomer component), and a monomer other than these (the third monomer component), the copolymerization ratio (first monomer component/second monomer component/third monomer component) is preferably 40 - 94 wt%/1 - 15 wt%/5 - 50 wt%, more preferably 50 - 89 wt%/1 - 10 wt%/10 - 40 wt%.

While the polymerization reaction may be performed by a method known per se and is not particularly limited, for example, a method including reacting the above-mentioned monomer in a solvent (e.g., ethyl acetate and the like) in the presence of a polymerization initiator (e.g., benzoyl peroxide, azobisisobutyronitrile and the like) at 50 - 70°C for 5 - 48 hr can be mentioned.

The acrylic polymer in the first aspect of the present invention is particularly preferably a 2-ethylhexylacrylate/acrylic acid/N-vinyl-2-pyrrolidone copolymer, a 2-ethylhexylacrylate/2-hydroxyethylacrylate/vinyl acetate copolymer, a 2-ethylhexylacrylate/acrylic acid copolymer and the like, more preferably a 2-ethylhexylacrylate/acrylic acid/N-vinyl-2-pyrrolidone copolymer.

While the glass transition temperature of the acrylic polymer in the first aspect of the present invention also varies depending on the copolymer composition, it is generally preferably -100 to -10°C, more preferably -90 to -20°C, from the aspect of adhesiveness of a patch preparation. The glass transition temperature is a measured value by a differential scanning calorimeter.

In the patch preparation (I) of the present invention, the content of the adhesive (adhesive polymer) in the adhesive layer is preferably 30 - 80 wt%, more preferably 40 - 70 wt%, in 100 wt% of the adhesive layer.

(iii) The lactic acid to be used in the first aspect of the present invention may be DL-lactic acid which is a racemate, or L-lactic acid or D-lactic acid which is an optically active substance. From the aspect of flowability, DL-lactic acid is preferable.

When the content of lactic acid in the adhesive layer is too low, the drug (i.e., compound A or a physiologically acceptable acid addition salt thereof) tends to show an insufficient skin permeability-promoting effect. When it is too high, skin irritation tends to increase. Hence, the content of lactic acid in the adhesive layer is preferably 0.1 - 10 wt% in 100 wt% of the adhesive layer. From the aspect of promotion of drug skin permeability, 1 - 10 wt% is more preferable and, in consideration of the physical skin irritation due to an increased adhesive force of the adhesive layer, 1 - 6 wt% is particularly preferable.

(iv) The sesame oil to be used in the first aspect of the present invention is an oil obtained by squeezing sesame seeds, which may be an oil derived from raw sesame seeds, an oil derived from roasted sesame seeds or a mixture of the both. It is possible to use "sesame oil", which is a commercially available product for food, medicine and the like.

When the content of the sesame oil in the adhesive layer is too low, a skin irritation-decreasing effect tends to be insufficient. When it is too high, sesame oil in the adhesive layer separates from the other components, and prevents production of a patch preparation. Therefore, the content of sesame oil in the adhesive layer is preferably 3 - 20 wt% in 100 wt% of the adhesive layer. Particularly, in consideration of various properties of the patch preparation and sufficient irritation-decreasing effect, the content is more preferably 4 - 18 wt%, further preferably 5 - 18 wt%, particularly preferably 10 - 16 wt%, in 100 wt% of the adhesive layer.

(vi) To further soften the adhesive layer, and reduce physical skin irritation during adhesion and/or detachment, the adhesive layer contains (vi) an organic liquid component. The "organic liquid component" in the first aspect of the present invention is an organic substance which itself is a liquid at room temperature (25°C), shows an action to plasticize the adhesive layer, and is compatible with an adhesive polymer constituting the above-mentioned adhesive. Sesame oil is a liquid at room temperature (25°C) and also has an action to plasticize the adhesive layer like the organic liquid component.

The organic liquid component includes fatty acid ester (hereinafter to be also abbreviated as "C8 - 18 (12 - 16)-C1 - 18 fatty acid ester") such as isopropyl myristate, ethyl laurate, isopropyl palmitate, ethyl oleate, isostearyl laurate, isotridecyl myristate, octyl palmitate and the like, which is formed from a fatty acid having a carbon number of 8 to 18 (preferably 12 - 16) and a monohydric alcohol having a carbon number of 1 to 18; fatty acid having a carbon number of 8 to 9 [for example, caprylic acid (octanoic acid, C8), pelargonic acid (nonanoic acid, C9) and the like]; glycerin fatty acid ester; glycols such as ethylene glycol, diethylene glycol, triethylene glycol, polyethylene glycol, 1,3-propanediol, polypropylene glycol and the like; fats and oils such as olive oil, castor oil, squalene and the like; organic solvent such as dimethyl sulfoxide, dimethylformamide, dimethylacetamide, dimethyllauryl amide, dodecyl pyrrolidone, isosorbitol, oleyl alcohol, lauric acid, N-methyl-2-pyrrolidone and the like; liquid surfactant such as polyoxyethylene alkyl ether sodium sulfate, polyoxyethylene lauryl ether sodium sulfate, sodium alkylnaphthalenesulfonate, polyoxyethyleneoleyl amine, polyoxyethylene oleyl ether sodium phosphate, polyoxyl stearate, decaglyceryl laurate, polyoxyethylene sorbitan monolaurate, polyoxyethylene sorbitan monostearate, sorbitan monolaurate, sorbitan trioleate, polyoxyethylenesorbitol tetraoleate, glycerol monooleate, sucrose ester of fatty acid, tocopherol and the like; hydrocarbons; plasticizer to be conventionally known such as phthalic acid ester and the like; further, ethoxylated stearyl alcohol, glycerol and the like. One kind alone or two or more kinds of these organic liquid components are used in combination. Among these, isopropyl myristate is particularly preferable.

The total content of the organic liquid component and sesame oil in the adhesive layer in the first aspect of the present invention is preferably 5 - 60 wt%, more preferably 10 - 50 wt%, in 100 wt% of the adhesive layer. When it is less than 5 wt%, a good, soft feeling of the adhesive layer sometimes cannot be achieved and a sufficient skin irritation-decreasing effect sometimes cannot be obtained. When it exceeds 60 wt%, the organic liquid component cannot be maintained in the adhesive even with the cohesive force of the adhesive, and blooms on the surface of the adhesive layer to decrease the adhesive force, thus increasing the possibility of falling off of the preparation from the skin surface during use by adhesion.

In the first aspect of the present invention, the content ratio of the sesame oil and organic liquid component (organic liquid component other than sesame oil) in the adhesive layer is preferably 1:1 - 10, more preferably 1:1 - 6, particularly preferably 1:1 - 3, by weight ratio (sesame oil:organic liquid component). When the content of the organic liquid component is lower than that of the sesame oil, the skin irritation-decreasing effect during detachment tends to become low. When the content of the organic liquid component exceeds 10 times that of the sesame oil, skin irritation (by lactic acid)-decreasing effect of the sesame oil tends to be difficult to obtain.

### (v) (v-1) Particular additive

A patch preparation generally has a large contacting area with the air. Therefore, a stabilizer is sometimes added to the adhesive layer to provide sufficient stability. In the present invention, it has been clarified that a desired stabilizing effect can be obtained by adding, as a substance that secures the stability of compound A or a physiologically acceptable acid addition salt thereof, and suppresses coloration of the patch preparation, the following particular additive, that is, 2-mercaptobenzimidazole, 2,6-di-tert-butyl-4-methylphenol and propyl gallate to the adhesive layer. Any one or more kinds thereof can be used. Of these, 2,6-di-tert-butyl-4-methylphenol (BHT) is preferable.

The content of the additive in the adhesive layer is preferably 0.3 - 10 wt% in 100 wt% of the adhesive layer. In consideration of the influence on the property of the adhesive layer, the content is more preferably 0.3 - 6 wt%, particularly preferably 0.3 - 2 wt%.

A crosslinking treatment can be applied to the adhesive layer. The crosslinking treatment is not particularly limited and can be performed by a means generally performed in the field, such as a chemical crosslinking treatment (crosslinking treatment using a crosslinking agent and the like), a physical crosslinking treatment (crosslinking treatment by irradiation of electron beam such as gamma ray, irradiation of ultraviolet light and the like) and the like. By the crosslinking treatment, the adhesive layer becomes a so-called adhesive layer in a gel state, which has suitable adhesiveness and cohesive force, while providing a soft feeling to the skin. In the patch preparation (I) of the present invention, when a chemical or physical crosslinking treatment is applied to the adhesive layer, stability of the drug tends to decrease during the production steps or preservation of the preparation (that is, the production amount of analogue tends to increase). It has been clarified in the present invention that the above-mentioned particular stabilizer enhances the stability of compound A or a physiologically acceptable acid addition salt thereof in the acrylic polymer that underwent the crosslinking treatment. Such crosslinking treatment is not essential and it is needless to say that even a patch preparation having an adhesive layer free of a crosslinking treatment can be naturally encompassed in the present invention.

When a chemical crosslinking treatment using a crosslinking agent is applied to the adhesive layer, crosslinking can be effectively caused by adding a crosslinking agent to the adhesive layer, though self-crosslinking can also be induced in the adhesive layer. The crosslinking agent is not particularly limited as long as it does not permit formation of crosslinking to be prevented by compound A or a physiologically acceptable acid addition salt thereof. Examples thereof include isocyanate compound, organometallic compound (for example, zirconium and zinc alaninate, zinc acetate, glycine ammonium zinc etc.), metal alcoholate (for example, tetraethyl titanate, tetraisopropyl titanate, aluminum isopropylate, aluminum sec-butyrate etc.), metal chelate compound (for example, dipropoxy bis(acetyl acetonate)titanate, tetraoctyleneglycol titanate, aluminum isopropylate, ethyl acetoacetate aluminum diisopropylate, aluminum tris(ethyl acetoacetate), aluminum tris(acetyl acetonate)) and the like. Particularly, when the adhesive layer contains an isocyanate compound, a decrease in the cohesive force of the adhesive layer during adhesion of the patch preparation to the human skin can be reduced, and cohesive failure does not easily occur during detachment of the adhesive layer. Thus, an isocyanate compound is preferable.

Examples of the isocyanate compound include aliphatic diisocyanate such as tetramethylene diisocyanate, hexamethylene diisocyanate and the like, alicyclic diisocyanate such as isophorone diisocyanate, hydrogenated xylylene diisocyanate, hydrogenated toluene diisocyanate, hydrogenated diphenylmethane diisocyanate and the like, aromatic ring-containing diisocyanate such as xylylene diisocyanate and the like, aromatic diisocyanate such as tolylenediisocyanate, 4,4'-diphenylmethane diisocyanate etc., and the like. One or more kinds of these may be used in a mixture.

While the amount of the crosslinking agent varies depending on the kind of crosslinking agent and adhesive polymer, it is generally preferably 0.01 - 10 wt%, more preferably 0.05 - 5 wt%, in 100 wt% of the adhesive layer. When it is less than 0.01 wt%, a sufficient cohesive force cannot be conferred to an adhesive layer since the crosslinking points are too few, which in turn may result in adhesive residue and strong skin irritation caused by cohesive failure during the detachment of the preparation from the skin. When it is more than 10 wt%, sufficient skin adhesion may not be afforded, though the cohesive force is high. In addition, skin irritation may occur due to the residual unreacted crosslinking agent. For example, the chemical crosslinking treatment can be carried out by, after addition of the crosslinking agent to the adhesive layer, steps of heating to not less than the crosslinking reaction temperature and preserving the layer, that is, an aging step. While the heating temperature therefor is appropriately determined according to the kind of the crosslinking agent, it is preferably 60 - 90°C, more preferably 60 - 80°C. The heating time is preferably 12 - 96 hr, more preferably 24 - 72 hr.

In the patch preparation (I) of the present invention, the adhesive layer is preferably a non-aqueous adhesive layer in view of the skin adhesion. The non-aqueous adhesive layer here is not necessarily limited to one completely free of water, but includes those containing a slight amount of water (e.g., less than 1 wt% in 100 wt% of an adhesive layer) derived from humidity in the air, skin and the like.

The adhesive layer in the first aspect of the present invention can contain a flavor, a colorant and other additives.

While the thickness of the adhesive layer is not particularly limited, it is preferably 20 - 300 µm, more preferably 30 - 300 µm, most preferably 50 - 300 µm. When the thickness of the adhesive layer is less than 20 µm, it may be difficult to obtain a sufficient adhesive force, and contain an effective amount of compound A or a physiologically acceptable acid addition salt thereof. When the thickness of the adhesive layer exceeds 300 µm, formation of the adhesive layer may become difficult (difficult coating). When the composition of the adhesive layer is the same, an adhesive layer free of a crosslinking treatment generally preferably has a thin thickness, whereas an adhesive layer subjected to a crosslinking treatment can have a high thickness.

The surface on the side opposite to the support side of the adhesive layer (adhesion surface to the skin) is preferably laminated with a release liner until actual use of the preparation. The release liner is not particularly limited, and a known release liner can be used. Specific examples thereof include a release liner wherein a release treating agent layer comprised of the release treating agent is formed on the surface of a substrate for a release liner, a plastic film having high releasability by itself, a release liner having a constitution wherein a release layer comprised of the aforementioned plastic film material having high releasability is formed on the surface of a substrate for a release liner and the like. The release surface of the release liner may be only one surface or both surfaces of the substrate.

In such release liner, the release treating agent is not particularly limited and, for example, release agents such as a long chain alkyl group-containing polymer, a silicone polymer (silicone release agent), a fluorine polymer (fluorine release agent) and the like can be mentioned. Examples of the substrate for a release liner include plastic films such as a polyethylene terephthalate (PET) film, a polyimide film, a polypropylene film, a polyethylene film, a polycarbonate film, a polyester (excluding PET) film and the like, and metallized plastic films wherein a metal is evaporated on these films; papers such as Japanese paper, Western paper, craft paper, glassine paper, fine paper and the like; a substrate made of a fibrous material such as non-woven fabric, cloth and the like; a metal foil and the like.

As the plastic film having high releasability by itself, polyethylene (low density polyethylene, linear low density polyethylene etc.), polypropylene, ethylene-α-olefin copolymers (block copolymer or random copolymer) such as ethylenepropylene copolymer and the like, a polyolefin film made of a polyolefin resin comprised of a mixture of these; Teflon (registered trade mark) film and the like can be used.

The release layer formed on the surface of the aforementioned substrate for a release liner can be formed by laminating or coating the aforementioned plastic film material having high releasability on the aforementioned substrate for a release liner.

While the thickness (total thickness) of the release liner is not particularly limited, it is generally not more than 200 µm, preferably 25 - 100 µm.

While the production method of the patch preparation (I) of the present invention is not particularly limited, it can be produced by, for example, the following production method.

First, an adhesive polymer including an acrylic polymer, compound A or a physiologically acceptable acid addition salt thereof, sesame oil, and one or more kinds of additives selected from the group consisting of 2-mercaptobenzimidazole, 2,6-di-tert-butyl-4-methylphenol and propyl gallate are added, together with an organic liquid component and other additives as necessary, to a suitable solvent and the mixture is sufficiently mixed until it becomes homogeneous. Examples of the solvent include ethyl acetate, toluene, hexane, 2-propanol, methanol, ethanol and the like. When a crosslinking agent is added, it is added to the mixture and the mixture is sufficiently mixed. Where necessary, a solvent may be added along with a crosslinking agent and they are mixed.

Then, the obtained mixture is applied to one surface of the support or a release treating surface of the release liner, and dried to form an adhesive layer. The aforementioned application can be performed by, for example, casting, printing and other techniques known per se to those of ordinary skill in the art. Thereafter, a release liner or support is adhered to the adhesive layer to form a laminate. When a crosslinking treatment is performed, the release liner or support is adhered to the adhesive layer, and they are left standing at 60 - 90°C, preferably 60 - 70°C, for 24 - 48 hr to promote the crosslinking reaction, whereby a crosslinked adhesive layer is formed.

Then, the release liner is peeled off, the exposed surface of the adhesive layer is immersed a lactic acid solution wherein lactic acid is dissolved in a solvent, and dried at about 40 - 100°C. After drying, a release-treated surface of a release liner different from the above is adhered to the adhesive layer.

The form of the patch preparation (I) of the present invention is not limited and may be, for example, tape-like, sheet-like and the like.

While the dose of the patch preparation (I) of the present invention varies depending on the age, body weight, symptom and the like of the patient, a patch preparation containing 0.1 - 50 mg of compound A is generally preferably adhered to 5 - 100 cm² of the skin of an adult at a frequency of 3 times a day - once a week.

The patch preparation (I) of the present invention enables transdermal administration of compound A to a patient by applying same to a skin of the patient. In addition, the patch preparation (I) of the present invention can treat a disease treatable by transdermal administration of compound A, by applying the patch preparation (I) to a skin of the patient with the disease. For example, schizophrenia can be treated by applying same to a skin of a patient with schizophrenia, and the like.

### 2. The second aspect of the present invention

(ii) 2- (4-Ethyl-1-piperazinyl) -4- (4-fluorophenyl) -5, 6, 7, 8, 9, 10-hexahydrocycloocta[b]pyridine (generic name "blonanserin") relating to the second aspect of the present invention is the same as compound A used in the first aspect of the present invention.

Compound A may be a free base or a physiologically acceptable acid addition salt thereof. Examples of acid addition salts with organic acid and inorganic acid include, but are not limited to, those similar to the first aspect of the present invention. Furthermore, compound A or a physiologically acceptable acid addition salt thereof may be any of solvate, hydrate and non-hydrate. Any one kind may be used alone or two or more kinds thereof may be used in combination.

Such compound A or a physiologically acceptable acid addition salt thereof is produced by a method similar to that used for the first aspect of the present invention. The produced compound A or a physiologically acceptable acid addition salt thereof may be pulverized as appropriate by a generally-used method.

While the content of the "compound A or a physiologically acceptable acid addition salt thereof " to be contained in the patch preparation (II) of the present invention needs to be determined in the same manner as in the first aspect of the present invention, and a preferable content can also be determined in the same manner.

The patch preparation (II) of the present invention contains a support and an adhesive layer formed on at least one surface of the support, wherein the adhesive layer contains, in addition to the above-mentioned (i) compound A or a physiologically acceptable acid addition salt thereof, the following (ii) acrylic polymer, (iii) lactic acid and (v-2) particular filler. Where necessary, it may have a release sheet on the surface opposite from the support side of the adhesive layer. The form of the patch preparation is not limited and may be, for example, tape-like, sheet-like and the like.

While the above-mentioned support is not particularly limited, it is preferably a material that does not permit compound A or a physiologically acceptable acid addition salt thereof, lactic acid, and the like in the adhesive layer to pass through the support and be lost from the back face thereof (face of the support on the side opposite from the side of the adhesive layer) to decrease the content thereof in the adhesive layer, that is, a material having impermeability to compound A or a physiologically acceptable acid addition salt thereof, lactic acid and the like. As mentioned below, in an embodiment wherein the adhesive layer contains an organic liquid component, sesame oil and the like, a material impermeable to compound A or a physiologically acceptable acid addition salt thereof, lactic acid, organic liquid component and sesame oil is preferable.

As the above-mentioned support satisfying the impermeability, those similar to the support used in the first aspect of the present invention can be used. Preferable embodiment thereof is also similar to that in the first aspect of the present invention.

As the porous film, those similar to the film used in the first aspect of the present invention can be used. Preferable embodiment thereof is also similar to that in the first aspect of the present invention.

The laminate film for the support is produced by a method similar to that used in the first aspect of the present invention.

The thickness of the support can be determined in the same manner as in the first aspect of the present invention.

(ii) The second aspect of the present invention contains an acrylic polymer as an adhesive in the adhesive layer. Compound A or a physiologically acceptable acid addition salt thereof shows high solubility in an acrylic polymer as compared to rubber polymers, and a small possibility of forming a crystal of compound A or a physiologically acceptable acid addition salt thereof in the adhesive layer. Therefore, an acrylic polymer is advantageous, and the adhesive is preferably constituted by an acrylic polymer alone. When an adhesive polymer other than an acrylic polymer is contained as the adhesive, the adhesive polymer other than an acrylic polymer is contained in not more than 10 wt% relative to the total weight of the adhesive in the adhesive layer (acrylic polymer is 90 wt% or more). Examples of the adhesive polymer other than an acrylic polymer include rubber adhesive polymers, silicone adhesive polymers and the like.

As the acrylic polymer in the second aspect of the present invention, an acrylic polymer comprising a unit of alkyl(meth)acrylate as a main constitution unit is preferable. The acrylic polymer comprising alkyl(meth)acrylate as a main constitution unit is preferably a copolymer of alkyl(meth)acrylate (the first monomer component) and a vinyl monomer having a functional group capable of being involved in a crosslinking reaction (the second monomer component), or a copolymer wherein a monomer other than these (the third monomer component) is further copolymerized, from the aspects of adhesiveness to human skin, solubility of drug during formulation of a preparation and the like.

Examples of the above-mentioned alkyl(meth)acrylate (the first monomer component) include those similar to the alkyl(meth)acrylate used in the first aspect of the present invention. Preferable embodiment thereof is also similar to that in the first aspect of the present invention.

Particularly preferable examples of alkyl(meth)acrylate (the first monomer component) include those similar to the alkyl(meth)acrylate used in the first aspect of the present invention. Preferable embodiment thereof is also similar to that in the first aspect of the present invention.

In the above-mentioned vinyl monomer having a functional group capable of being involved in a crosslinking reaction (the second monomer component), as the functional group capable of being involved in a crosslinking reaction, those similar to the functional group used in the first aspect of the present invention can be used. Preferable embodiment thereof is also similar to that in the first aspect of the present invention.

In addition, the above-mentioned other monomer (the third monomer component) is mainly used for adjusting the cohesive force of the adhesive layer, adjusting solubility and releasability of a drug (compound A or a physiologically acceptable acid addition salt thereof) and the like. Examples of the monomer (the third monomer component) include those similar to the monomers exemplified in the first aspect of the present invention. Preferable embodiment thereof is also similar to that in the first aspect of the present invention.

When the acrylic polymer is a copolymer of alkyl(meth)acrylate (the first monomer component) and a vinyl monomer having a functional group capable of being involved in a crosslinking reaction (the second monomer component), the copolymerization ratio (first monomer component/second monomer component) is determined in the same manner as in the first aspect of the present invention, and a preferable range thereof is also similar to that in the first aspect of the present invention.

When the acrylic polymer is a copolymer of alkyl(meth)acrylate (the first monomer component), a vinyl monomer having a functional group capable of being involved in a crosslinking reaction (the second monomer component), and a monomer other than these (the third monomer component), the copolymerization ratio (first monomer component/second monomer component/third monomer component) is determined in the same manner as in the first aspect of the present invention, and a preferable range thereof is also similar to that in the first aspect of the present invention.

While the polymerization reaction may be performed by a method known per se and is not particularly limited, for example, a method including reacting the above-mentioned monomer in a solvent (e.g., ethyl acetate and the like) in the presence of a polymerization initiator (e.g., benzoyl peroxide, azobisisobutyronitrile and the like) at 50 - 70°C for 5 - 48 hr can be mentioned.

As a particularly preferable acrylic polymer in the second aspect of the present invention, those similar to the acrylic polymer used in the first aspect of the present invention can be used.

The glass transition temperature of the acrylic polymer in the second aspect of the present invention is determined in the same manner as in the first aspect of the present invention, and a preferable range thereof is similar to that in the first aspect of the present invention.

In the patch preparation (II) of the present invention, the content of the adhesive (adhesive polymer such as acrylic polymer and the like) in the adhesive layer is the balance after removing (i) compound A or a physiologically acceptable acid addition salt thereof, (iii) lactic acid and (v-2) a particular filler, and the following various formulation components themselves to be added as necessary. The content is an amount necessary for the completion of the adhesive layer, which is, for example, preferably 20 - 90 wt%, more preferably 30 - 80 wt%, in 100 wt% of the adhesive layer.

(iii) The lactic acid contained in the adhesive layer may be any as long as it is generally used in the technical field. It may be DL-lactic acid which is a racemate, or L-lactic acid or D-lactic acid which is an optically active substance. From the aspect of easy availability, L-lactic acid or DL-lactic acid is preferable. Particularly, from the aspect of flowability, DL-lactic acid is preferable. While the content of the lactic acid in the adhesive layer can be appropriately determined and is not particularly limited, it is preferably 0.1 - 10 wt% in 100 wt% of the adhesive layer. When it is less than 0.1 wt%, an effective amount of the drug may not be transferred into the blood. When it exceeds 10 wt%, the cohesive force of the adhesive layer may decrease. In consideration of the influence on the skin irritation, lactic acid is more preferably used at not more than 6 wt%, further preferably not more than 5 wt%. Specifically, 0.1 - 6 wt% is more preferable, and 0.1 - 5 wt% is further preferable.

(v-2) Particular fillers in the second aspect of the present invention are magnesium aluminometasilicate, polyvinylpyrrolidone and an aminoalkyl methacrylate copolymer. Any one kind of these may be used alone or two or more kinds thereof may be used in combination. The addition of such particular filler can suppress a decrease in the adhesiveness in the presence of water. The content of the filler in the adhesive layer is preferably 0.1 - 40 wt%, more preferably 0.1 - 25 wt%, further preferably 0.5 - 5 wt%, in 100 wt% of the adhesive layer. Particularly, the content of magnesium aluminometasilicate and/or an aminoalkyl methacrylate copolymer is particularly preferably 0.5 - 2.4 wt%. When it is less than 0.1 wt%, a sufficient adhesiveness-improving effect of the patch preparation in the presence of water tends to be difficult to obtain, and when it exceeds 40 wt%, the patch preparation may not show sufficient adhesiveness. Among magnesium aluminometasilicate, polyvinylpyrrolidone and an aminoalkyl methacrylate copolymer, magnesium aluminometasilicate is preferable, since it can improve the adhesiveness of the patch preparation in the presence of water with a comparatively small amount of addition.

Magnesium aluminometasilicate is available under the trade name of, for example, Neusilin from Fuji Chemical Industry. In addition, magnesium aluminometasilicate is preferably an amorphous composite oxide of aluminum, magnesium and silicon atom, which are three-dimensionally polymerized via an oxygen atom. Such composite oxide is more specifically magnesium aluminometasilicate represented by the formula: Al₂O_{3/}aMgO/bSiO₂·nH₂O wherein a=0.3 - 3 and b=0.3 - 5. Due to its porous structure, such magnesium aluminometasilicate is considered to act more advantageously when the adhesiveness in the presence of water is improved.

Polyvinylpyrrolidone is a nonionic water-soluble polymer compound wherein N-vinyl-2-pyrrolidone is polymerized under given conditions. While the molecular weight of polyvinylpyrrolidone is not particularly limited, a weight average molecular weight thereof is preferably about 2,000 - 1,500,000. In addition, while the particle size thereof is not particularly limited, an average particle size thereof is preferably 15 - 350 µm.

Polyvinylpyrrolidone can be a commercially available product, and is commercially available under the trade names of Kollidon K90, Kollidon 12PF, Kollidon 17PF, Kollidon 25, Kollidon 30, Kollidon 90F (BASF Japan Ltd.) and the like.

The aminoalkyl methacrylate copolymer is a fine particles of a copolymer of methylmethacrylate (15 - 35 wt%), butylmethacrylate (15 - 35 wt%) and dimethylaminoethylmethacrylate (30 - 70 wt%) (weight average molecular weight of about 75,000 - 300,000). While the particle size is not particularly limited, an average particle size thereof is generally 10 - 100 µm.

Such aminoalkyl methacrylate copolymer can be a commercially available product and is commercially available under the trade names of, for example, EUDRAGIT (registered trade mark) EPO (manufactured by Evonic-Degussa) which is a fine particle-type of EUDRAGIT (registered trade mark) E100, EUDRAGIT (registered trade mark) E125 and the like.

(vi) In the patch preparation (II) of the present invention, the adhesive layer can contain an organic liquid component from the aspects of conferring of a soft feeling to the adhesive layer, reduction of pain and irritation (physical irritation) due to skin adhesive force when a patch preparation is detached from the skin and the like.

As such organic liquid component, those similar to the organic liquid component used in the first aspect of the present invention can be used. Preferable embodiment thereof is also similar to that in the first aspect of the present invention.

The adhesive layer may contain (iv) sesame oil. Sesame oil is liquid at 25°C, and also has an action to plasticize an adhesive layer, as does the organic liquid component. Sesame oil may be an oil derived from raw sesame seeds, an oil derived from roasted sesame seeds or a mixture of the both. It is possible to use "sesame oil", which is a commercially available product for food, medicine and the like.

In the second aspect of the present invention, the content of the organic liquid component in an adhesive layer is preferably 5 - 60 wt%, more preferably 10 - 50 wt%, in 100 wt% of the adhesive layer. When it is less than 5 wt%, a good, soft feeling of the adhesive layer sometimes cannot be achieved and a sufficient skin irritation-decreasing effect sometimes cannot be obtained, due to insufficient plasticization of the adhesive layer. Conversely, when it exceeds 60 wt%, the organic liquid component cannot be maintained in the adhesive even with the cohesive force of the adhesive, and blooms on the surface of the adhesive layer to make the adhesive force too weak, thus increasing the possibility of falling off of the preparation from the skin surface during use by adhesion. When sesame oil is used along with C8 - 18(12 - 16)-C1 - 18 fatty acid ester, the C8 - 18(12 - 16)-C1 - 18 fatty acid ester is contained at preferably 2 - 40 wt%, more preferably 7 - 30 wt%, in 100 wt% of the adhesive layer, and sesame oil is preferably contained at 3 - 20 wt% in 100 wt% of the adhesive layer.

In the patch preparation (II) of the present invention, the adhesive layer can contain (v-1) a stabilizer (additive). Here, the stabilizer is a substance that secures the stability of compound A or a physiologically acceptable acid addition salt thereof, and suppresses coloration of the patch preparation. Examples thereof include 2-mercaptobenzimidazole, 2,6-di-tert-butyl-4-methylphenol, propyl gallate and the like. Any one or more kinds thereof can be used. Of these, 2,6-di-tert-butyl-4-methylphenol (BHT) is preferable.

The content of the stabilizer in the adhesive layer is preferably 0.3 - 10 wt% in 100 wt% of the adhesive layer. Particularly, in consideration of the influence on the property of the adhesive layer, the content is more preferably 0.3 - 6 wt%, particularly preferably 0.3 - 2 wt%, in 100 wt% of the adhesive layer.

In the patch preparation (II) of the present invention, a crosslinking treatment can be applied to the adhesive layer by a chemical crosslinking treatment (crosslinking treatment using a crosslinking agent and the like), a physical crosslinking treatment (crosslinking treatment by irradiation of electron beam such as gamma ray, irradiation of ultraviolet light and the like) and the like. The crosslinking treatment can be performed by a means generally used in the field. The crosslinking treatment is preferably a chemical crosslinking treatment using a crosslinking agent, since it does not easily exert an adverse influence on compound A.

When a chemical crosslinking treatment using a crosslinking agent is applied to the adhesive layer, crosslinking can be effectively caused by adding a crosslinking agent to the adhesive layer, though self-crosslinking can also be induced in the adhesive layer. Examples of the crosslinking agent include those similar to the crosslinking agent used in the first aspect of the present invention. Preferable embodiment thereof is also similar to that in the first aspect of the present invention.

The amount of the crosslinking agent is determined in the same manner as in the first aspect of the present invention, and a preferable amount thereof is also determined in the same manner as in the first aspect of the present invention. The chemical crosslinking treatment can be performed according to a method similar to that in the first aspect of the present invention. Preferable embodiment thereof is also similar to that in the first aspect of the present invention.

In the patch preparation (II) of the present invention, the thickness of the adhesive layer is preferably 20 - 300 µm, more preferably 30 - 300 µm, most preferably 50 - 300 µm. When the thickness of the adhesive layer is less than 20 µm, it may be difficult to obtain a sufficient adhesive force, and contain an effective amount of compound A or a physiologically acceptable acid addition salt thereof. When the thickness of the adhesive layer exceeds 300 µm, formation of the adhesive layer may become difficult (difficult coating).

In the patch preparation (II) of the present invention, the adhesive layer is preferably a nonaqueous adhesive layer from the aspects of the skin adhesion. The non-aqueous adhesive layer here is not necessarily limited to one completely free of water, but includes those containing a slight amount of water (e.g., less than 1 wt% of the total weight of an adhesive layer) derived from humidity in the air, skin and the like.

The patch preparation (II) of the present invention comprises a support and an adhesive layer, and is preferably provided with a release liner.

As the release liner, one similar to the release liner used in the first aspect of the present invention can be used.

As a release treating agent for such release liner, one similar to the release treating agent used in the first aspect of the present invention can be used. As a substrate for the release liner, one similar to the substrate used in the first aspect of the present invention can be used.

While the thickness (total thickness) of the release liner is not particularly limited, it is generally not more than 200 µm, preferably 25 - 100 µm.

While the production method of the patch preparation (II) of the present invention is not particularly limited, it can be produced by, for example, the following production method.

First, an adhesive polymer, compound A or a physiologically acceptable acid addition salt thereof, and a filler are added, together with an organic liquid component and other additives as necessary, to a suitable solvent and the mixture is sufficiently mixed until it becomes homogeneous. Examples of the solvent include ethyl acetate, toluene, hexane, 2-propanol, methanol, ethanol and the like. When a crosslinking agent is added, it is added to the mixture and the mixture is sufficiently mixed. Where necessary, a solvent may be added along with a crosslinking agent and they are mixed.

Then, the obtained mixture is applied to one surface of the support or a release treating surface of the release liner, and dried to form an adhesive layer. The aforementioned application can be performed by, for example, casting, printing and other techniques known per se to those of ordinary skill in the art. Thereafter, a release liner or support is adhered to the adhesive layer to form a laminate. When a crosslinking treatment is performed, the release liner or support is adhered to the adhesive layer, and they are left standing at 60 - 90°C, preferably 60 - 70°C, for 24 - 48 hr to promote the crosslinking reaction, whereby a crosslinked adhesive layer is formed.

Then, the release liner is peeled off, the exposed surface of the adhesive layer is immersed a lactic acid solution wherein lactic acid is dissolved in a solvent, and dried at about 40 - 100°C. After drying, a release-treated surface of a release liner different from the above is adhered to the adhesive layer.

The dose of the patch preparation (II) of the present invention is determined in the same manner as in the first aspect of the present invention.

The patch preparation (II) of the present invention enables transdermal administration of compound A to a patient by applying same to a skin of the patient. In addition, the patch preparation (II) of the present invention can treat a disease treatable by transdermal administration of compound A, by applying the patch preparation (I) to a skin of the patient with the disease. For example, schizophrenia can be treated by applying same to a skin of a patient with schizophrenia, and the like.

### Examples

The first and the second aspects of the present invention are explained in detail in the following by referring to Examples, which are not to be construed as limitative. In the following sentences, "parts" and "%" all mean parts by weight and wt%, unless particularly specified.

Examples 1A - 10A and Comparative Examples 1A - 9A recited below relate to the first aspect of the present invention.

### [Preparation of acrylic polymer A]

Under an inert gas atmosphere, 2-ethylhexylacrylate (75 parts), N-vinyl-2-pyrrolidone (22 parts), acrylic acid (3 parts) and azobisisobutyronitrile (0.2 part) were subjected to solution polymerization in ethyl acetate at 60°C to give an acrylic copolymer (acrylic polymer A) solution. The glass transition point of the acrylic copolymer (acrylic polymer A) was -45.2°C.

### [Example 1A]

Acrylic polymer A (57.09 parts), compound A (6.45 parts), isopropyl myristate (hereinafter to be referred to as "IPM") (17.73 parts), sesame oil (14.51 parts), and 2,6-di-tert-butyl-4-methylphenol (hereinafter to be referred to as "BHT") (0.97 part) were dissolved in a moderate amount of ethyl acetate and the solution was sufficiently mixed until it became homogeneous. As a crosslinking agent, trifunctional isocyanate (CORONATE HL (manufactured by Japan Polyurethane Industry), 0.26 part) was added. The concentration of the base was adjusted to 20 wt% with ethyl acetate and the mixture was sufficiently mixed and stirred until it became homogeneous to give a coating solution. The obtained coating solution was applied to a release-treated surface of a release liner, which was a 75 µm-thick polyethylene terephthalate (hereinafter to be referred to as "PET") film subjected to a release treatment with a silicone release agent, such that the thickness of the adhesive layer after drying was about 120 µm, and dried to form an adhesive layer. The adhesive surface of the adhesive layer thus formed was adhered to a nonwoven fabric side of a laminate film of a 3.5 µm-thick PET film and a PET nonwoven fabric with a fabric weight of 12 g/m² to give a laminate. The laminate was left standing at 70°C for 48 hr to prepare a crosslinked adhesive layer. After standing, the aforementioned release liner was detached, the adhesive surface of the adhesive layer was immersed in a solution of DL-lactic acid in ethyl acetate (lactic acid:ethyl acetate=1:2 (weight ratio)), and dried such that the amount of lactic acid in the adhesive layer after drying was 2.99 parts relative to the aforementioned crosslinked adhesive layer (97.01 parts) (lactic acid content per total weight of the adhesive layer was 2.99%). After drying, a release liner subjected to the same release treatment as above was separately prepared, and adhered to the release-treated surface thereof to give a patch preparation of Example 1A.

The above-mentioned base concentration refers to a value (wt%) obtained by subtracting the weight (g) of ethyl acetate from the weight (g) of the coating solution, dividing the obtained value by the weight (g) of the coating solution and multiplying the obtained value by 100.

### [Example 2A]

In the same manner as in Example 1A except that acrylic polymer A (57.08 parts), IPM (22.57 parts), and sesame oil (9.68 parts) were used, the patch preparation of Example 2A was obtained.

### [Example 3A]

In the same manner as in Example 1A except that acrylic polymer A (57.08 parts), IPM (27.41 parts), and sesame oil (4.84 parts) were used, the patch preparation of Example 3A was obtained.

### [Example 4A]

In the same manner as in Example 1A except that compound A (6.52 parts), acrylic polymer A (57.68 parts), IPM (17.91 parts), sesame oil (14.66 parts), lactic acid (1.99 parts), and BHT (0.98 parts) were used, the patch preparation of Example 4A was obtained.

### [Example 5A]

In the same manner as in Example 1A except that compound A (6.25 parts), acrylic polymer A (55.32 parts), IPM (17.19 parts), sesame oil (14.06 parts), lactic acid (5.99 parts), BHT (0.94 part), and a crosslinking agent (0.25 part) were used, the patch preparation of Example 5A was obtained.

### [Comparative Example 1A]

In the same manner as in Example 1A except that sesame oil was not used, and instead, IPM (32.25 parts) and acrylic polymer A (57.08 parts) were used, the patch preparation of Comparative Example 1A was obtained.

### [Comparative Example 2A]

In the same manner as in Comparative Example 1A except that compound A (6.52 parts), acrylic polymer A (57.67 parts), IPM (32.58 parts), lactic acid (1.99 parts), and BHT (0.98 part) were used, the patch preparation of Comparative Example 2A was obtained.

### [Comparative Example 3A]

In the same manner as in Comparative Example 1A except that compound A (6.25 parts), acrylic polymer A (55.32 parts), IPM (31.25 parts), lactic acid (5.99 parts), BHT (0.94 part), and a crosslinking agent (0.25 part) were used, the patch preparation of Comparative Example 3A was obtained.

### [Comparative Example 4A]

In the same manner as in Comparative Example 1A except that lactic acid was not used, and compound A (6.65 parts), acrylic polymer A (58.85 parts), IPM (33.24 parts), and BHT (1.00 part) were used, the patch preparation of Comparative Example 4A was obtained.

### [Comparative Example 5A]

In the same manner as in Comparative Example 1A except that sesame oil was not used, and instead, linoleic acid (14.51 parts) and acrylic polymer A (57.09 part) were used, the patch preparation of Comparative Example 5A was obtained.

### <Evaluation test>

The preparations produced in the above-mentioned Examples and Comparative Examples were subjected to the following tests. The results thereof are shown in Table 1.

### (Experimental Example 1: Transdermal absorption experiment)

Male 6-week-old SD rats were anesthetized and the back was shaved. The preparation was cut into a 2.5 cm × 5 cm tape preparation, and adhered to the shaved part on the back of the rat. Blood samples were collected over time and the serum was separated. The obtained serum was quantified by high performance liquid chromatography (hereinafter to be referred to as HPLC), and the concentration of compound A in the serum was measured 24 hr after administration.

### (Experimental Example 2: Cumulative irritation test by repetitive administration for 3 days)

A rabbit (about 17-week-old, body weight 3 kg) after cutting the hair on the back was purchased and, after 1 week quarantine, an Elizabethan collar was set. The next day, the back of the rabbit was shaved with a T-shaped razor, and the test was started 3 days later. The preparation was cut into a 1.5 cm×1.5 cm tape preparation, and adhered to the normal skin on the back of the rat. A non-woven fabric adhesive bandage (MESHPORE, manufactured by NICHIBAN Co., Ltd.) was applied to cover the tape and, after occlusive dressing for 24 hr, the tape preparation was removed and the rabbit was left for about 30 min. Again, a new tape preparation with the same size was adhered to a similar position, and an operation of covering, occlusive dressing for 24 hr, removing, and leaving for about 30 min was performed. A similar operation was performed once again. After the aforementioned operation, the administration area was visually observed, and the skin reaction was evaluated according to the diagnostic criteria of the Draize method shown below.

### (Diagnostic criteria)

- erythema
   0: no erythema
   1: extremely slight erythema
   2: clear erythema
   3: medium-strong erythema
   4: strong erythema - scabbing
- edema formation
   0: no edema
   1: extremely slight edema
   2: clear edema
   3: moderate edema
   4: strong edema

### (Experimental Example 3: Adhesive strength test)

A sample cut into width 24 mm, length 50 mm was press-adhered to a stainless board by one reciprocation of a 2 kg roller and, after 30 min, the sample was peeled off at a peel angle 180°, rate 300 mm/min and the release force at that time was measured. The test was performed with n=3, the load at 3 points was measured for each test and the 9 points in total were averaged. The test points were 20, 40, 60 mm from the detaching start position.

**Table 1**

| | compound A (parts) | acrylic polymer A (parts) | IPM (parts) | sesame oil (parts) | linoleic acid (parts) | lactic acid (parts) | BHT (parts) | crosslinking agent (parts) | blood concentration (ng/mL) after 24 hr | skin irritation erythema+ edema | adhesive force (N/24 mm) |
|---|---|---|---|---|---|---|---|---|---|---|---|
| Ex. 1A | 6.45 | 57.09 | 17.73 | 14.51 | 0 | 2.99 | 0.97 | 0.26 | 29 | 3.0 | 1.96 |
| Ex. 2A | 6.45 | 57.08 | 22.57 | 9.68 | 0 | 2.99 | 0.97 | 0.26 | 38 | 3.0 | 1.77 |
| Ex. 3A | 6.45 | 57.08 | 27.41 | 4.84 | 0 | 2.99 | 0.97 | 0.26 | 31 | 3.3 | 1.75 |
| Ex. 4A | 6.52 | 57.68 | 17.91 | 14.66 | 0 | 1.99 | 0.98 | 0.26 | 27 | 2.5 | 1.93 |
| Ex. 5A | 6.25 | 55.32 | 17.19 | 14.06 | 0 | 5.99 | 0.94 | 0.25 | 35 | 2.8 | 2.36 |
| Com. Ex. 1A | 6.45 | 57.08 | 32.25 | 0 | 0 | 2.99 | 0.97 | 0.26 | 29 | 3.5 | 1.49 |
| Com. Ex. 2A | 6.52 | 57.67 | 32.58 | 0 | 0 | 1.99 | 0.98 | 0.26 | 32 | 3.8 | 1.64 |
| Com. Ex. 3A | 6.25 | 55.32 | 31.25 | 0 | 0 | 5.99 | 0.94 | 0.25 | 34 | 4.3 | 2.04 |
| Com. Ex. 4A | 6.65 | 58.85 | 33.24 | 0 | 0 | 0 | 1.00 | 0.26 | 12 | 2.3 | 2.33 |
| Com. Ex. 5A | 6.45 | 57.09 | 17.73 | 0 | 14.51 | 2.99 | 0.97 | 0.26 | 17 | 3.0 | 1.63 |

The following are clear from Table 1.
(1) In Comparative Examples 1A - 3A without containing sesame oil in an adhesive layer, the development of skin irritation became remarkable as the content of lactic acid in the adhesive layer increased. In contrast, in Examples 1A - 5A containing sesame oil in an adhesive layer, the development of skin irritation was suppressed irrespective of the high or low content of lactic acid. In Comparative Example 4A without containing lactic acid and sesame oil, the blood concentration of compound A could not be maintained. In Comparative Example 5A containing linoleic acid instead of sesame oil, the development of skin irritation was suppressed, but the blood concentration could not be maintained.
(2) It is clear that an adhesive force of the preparation became high as the content of sesame oil in the adhesive layer increased.

### (Experimental Example 4: Preparation stability test)

Preparation samples each containing the stabilizer described in Table 2 were produced according to the following formulations, and the stability of the preparations was evaluated based on the production rate of an analogue in the preparations due to preservation and coloration of the preparations. Measurement of the production rate of an analogue and evaluation of the coloration of the preparations were performed by the following methods.

### [Pharmaceutical formulation]

Acrylic polymer A (53.00 parts), compound A (6.00 parts), IPM (29.90 parts), and stabilizer (0.90 part) were dissolved in a moderate amount of ethyl acetate and the solution was sufficiently mixed until it became homogeneous. As a crosslinking agent, trifunctional isocyanate (CORONATE HL (manufactured by Japan Polyurethane Industry), 0.20 part) was added. The concentration of the base was adjusted to 20 wt% with ethyl acetate and the mixture was sufficiently mixed and stirred until it became homogeneous to give a coating solution. The obtained coating solution was applied to a release-treated one surface of a release liner, which was a 75 µm-thick PET film subjected to a release treatment with a silicone release agent, such that the thickness of the adhesive layer after drying was about 60 µm, and dried to form an adhesive layer. The adhesive surface of the adhesive layer thus formed was adhered to a nonwoven fabric side of a laminate film of a 3.5 µm-thick PET film and a PET nonwoven fabric with a fabric weight of 12 g/m² to give a laminate. The laminate was left standing at 70°C for 48 hr to prepare a crosslinked adhesive layer. After standing, the aforementioned release liner was detached, the exposed surface of the adhesive layer was immersed in a solution of lactic acid in ethyl acetate (lactic acid:ethyl acetate=1:2 (weight ratio)), and dried such that the amount of lactic acid in the adhesive layer after drying was 10.00 parts relative to the aforementioned crosslinked adhesive layer (90.00 parts) (lactic acid content per total weight of the adhesive layer was 10.00%). After drying, a release liner subjected to a release treatment was separately prepared, and adhered to the release-treated surface thereof to give a patch preparation.

### [Analogue production rate]

A patch preparation was preserved at 70°C for 1 week, and the drug in the patch preparation was extracted with methanol, analyzed by HPLC, and the production rate of the total analogue of the drug was examined. The production rate of the total analogue of the drug was calculated in area % from the HPLC chart and according to the formula: [(total peak area corresponding to analogue of drug)/(peak area corresponding to drug)] × 100.

### [Coloration]

A patch preparation was preserved at 70°C for 1 week, and the color of the patch preparation was evaluated by visual observation.

**Table 2**

| | rate of total analogue (area %) | color |
|---|---|---|
| BHT | 0.69 | none |
| 2-mercaptobenzimidazole | 0.79 | none |
| propyl gallate | 0.84 | none |
| benzotriazole | 1.64 | yellow |
| L-ascorbic acid | 1.22 | brown |
| sodium edetate | 1.24 | yellow |
| none | 1.20 | yellow |

From the results of Table 2, it is clear that BHT, 2-mercaptobenzimidazole and propyl gallate show remarkable suppressive effects on the production of the total analogue of compound A and coloration of the preparation, and stabilize a compound A-containing patch preparation. In contrast, benzotriazole, L-ascorbic acid and sodium edetate did not show a stabilizing effect on a compound A-containing patch preparation, though they are used comparatively often as stabilizers for patch preparations.

### [Example 6A] - [Example 10A] and [Comparative Example 6A] - [Comparative Example 9A]

In the same manner as in Example 1A except that the compositions shown in the following Table 3 were employed, the patch preparations of [Example 6A] - [Example 10A] and [Comparative Example 6A] - [Comparative Example 9A] were obtained.

In the Table, 2-MBI means 2-mercaptobenzimidazole.

**Table 3**

| | compound A (parts) | acrylic polymer A (parts) | IPM (parts) | sesame oil (parts) | linoleic acid (parts) | lactic acid (parts) | BHT (parts) | 2-MBI (parts) | propyl gallate (parts) | benzotriazole (parts) | L-ascorbic acid (parts) | sodium edetate (parts) | crosslink-king agent (parts) |
|---|---|---|---|---|---|---|---|---|---|---|---|---|---|
| Ex. 6A | 6.45 | 57.09 | 17.73 | 14.51 | 0 | 2.99 | 0 | 0.97 | 0 | 0 | 0 | 0 | 0.26 |
| Ex. 7A | 6.45 | 57.09 | 17.73 | 14.51 | 0 | 2.99 | 0 | 0 | 0.97 | 0 | 0 | 0 | 0.26 |
| Com. Ex. 6A | 6.45 | 57.09 | 17.73 | 14.51 | 0 | 2.99 | 0 | 0 | 0 | 0.97 | 0 | 0 | 0.26 |
| Com. Ex. 7A | 6.45 | 57.09 | 17.73 | 14.51 | 0 | 2.99 | 0 | 0 | 0 | 0 | 0.97 | 0 | 0.26 |
| Com. Ex. 8A | 6.45 | 57.09 | 17.73 | 14.51 | 0 | 2.99 | 0 | 0 | 0 | 0 | 0 | 0.97 | 0.26 |
| Com. Ex. 9A | 6.45 | 58.06 | 17.73 | 14.51 | 0 | 2.99 | 0 | 0 | 0 | 0 | 0 | 0 | 0.26 |
| Ex. 8A | 6.45 | 57.76 | 17.73 | 14.51 | 0 | 2.99 | 0.3 | 0 | 0 | 0 | 0 | 0 | 0.26 |
| Ex. 9A | 6.45 | 56.06 | 17.73 | 14.51 | 0 | 2.99 | 2 | 0 | 0 | 0 | 0 | 0 | 0.26 |
| Ex. 10A | 6.45 | 53.06 | 17.73 | 14.51 | 0 | 2.99 | 5 | 0 | 0 | 0 | 0 | 0 | 0.26 |

The calculation of the production rate of the analogue of the patch preparations of [Example 6A] - [Example 10A] and [Comparative Example 6A] - [Comparative Example 9A], and evaluation of coloration thereof were performed according to the methods similar to the aforementioned methods. The results thereof are shown in Table 4.

**[Table 4]**

| | rate of total analogue (area %) | color |
|---|---|---|
| Ex. 6A | 0.66 | none |
| Ex. 7A | 0.71 | none |
| Com. Ex. 6A | 1.38 | yellow |
| Com. Ex. 7A | 1.03 | brown |
| Com. Ex. 8A | 1.04 | yellow |
| Com. Ex. 9A | 1.37 | yellow |
| Ex. 8A | 0.67 | none |
| Ex. 9A | 0.64 | none |
| Ex. 10A | 0.55 | none |

As shown in Examples 6A, 7A, it is clear that 2-mercaptobenzimidazole and propyl gallate show remarkable suppressive effects on the production of the total analogue of compound A and coloration of the preparation, and stabilize a compound A-containing patch preparation. In contrast, as shown in Comparative Examples 6A - 8A, benzotriazole, L-ascorbic acid and sodium edetate did not show a stabilizing effect on a compound A-containing patch preparation.

As shown in Examples 8A - 10A, it was confirmed in patch preparations wherein the content of BHT in the adhesive layer was changed that the amount of BHT of 0.3 - 5 wt% in 100 wt% of the adhesive layer showed remarkable suppressive effects on the production of the total analogue of compound A and coloration of the preparation, and stabilized a compound A-containing patch preparation.

Examples 1B - 15B and Comparative Examples 1B - 3B recited below relate to the second aspect of the present invention.

### [Preparation of acrylic polymer A]

In the same manner as in the Examples relating to the first aspect of the present invention, an acrylic polymer A solution was prepared.

### [Example 1B]

To acrylic polymer A (55.94 parts), compound A (6.46 parts), isopropyl myristate(hereinafter to be referred to as "IPM") (17.77 parts), sesame oil (14.54 parts), 2,6-di-tert-butyl-4-methylphenol (hereinafter to be referred to as "BHT") (0.97 parts), and magnesium aluminometasilicate (Neusilin (trade name) type: UFL2, manufactured by Fuji Chemical Industry) (1.26 parts) was added a moderate amount of ethyl acetate and the solution was sufficiently mixed until it became homogeneous. As a crosslinking agent, trifunctional isocyanate (CORONATE HL (manufactured by Japan Polyurethane Industry), 0.06 part) was added. The concentration of the base was adjusted to 24 wt% with ethyl acetate and the mixture was sufficiently mixed and stirred until it became homogeneous to give a coating solution. The obtained coating solution was applied to a release-treated surface of a release liner, which was a 75 µm-thick polyethylene terephthalate (hereinafter "PET") film subjected to a release treatment with a silicone release agent, such that the thickness of the adhesive layer after drying was 120 µm, and dried to form an adhesive layer. The adhesive surface of the adhesive layer thus formed was adhered to a nonwoven fabric side of a laminate support of a 3.5 µm-thick PET film and a PET nonwoven fabric with a fabric weight of 12 g/m² to give a laminate. The laminate was left standing at 70°C for 48 hr to prepare a laminate having a crosslinked adhesive layer. After standing, the release liner of the laminate having a crosslinked adhesive layer was detached, the crosslinked adhesive layer was immersed in lactic acid such that the final content of lactic acid was 3 parts relative to the crosslinked adhesive layer (97 parts). After drying, a release liner which was the same as the above-mentioned release liner was separately prepared, and adhered to the adhesive surface of the crosslinked adhesive layer to give a patch preparation of Example 1B.

The above-mentioned base concentration (w%) refers to a value (wt%) obtained by subtracting the weight (g) of ethyl acetate from the weight (g) of the coating solution, dividing the obtained value by the weight (g) of the coating solution and multiplying the obtained value by 100.

### [Example 2B] - [Example 11B] and [Comparative Example 1B] - [Comparative Example 3B]

In the same manner as in Example 1B except that the compositions and the thickness of the adhesive layer shown in the following Table 5 were employed, the patch preparations of [Example 2B] - [Example 11B] and [Comparative Example 1B] - [Comparative Example 3B] were obtained.

In the Table, PVP shows polyvinylpyrrolidone, Kollidon (registered trade mark) K90 (K value = 90, weight average molecular weight about 1,250,000) (manufactured by BASF), and EPO shows EUDRAGIT (registered trade mark) EPO (manufactured by Evonic-Degussa) which is a fine particle type of aminoalkyl methacrylate copolymer, EUDRAGIT (registered trade mark) E100.

### <Experimental Example>

### [Adhesive force measurement test]

The Example was performed by the method similar to that in the Examples relating to the first aspect of the present invention. The results thereof are shown in Table 5.

### [Holding power measurement test]

A sample is cut in 10 mm, length 50 mm and one end (about 25 mm) thereof is pressed against a bakelite (phenol resin) plate by one reciprocation of a roller (weight 850 g). The other end is reinforced with an auxiliary sheet. This is set on a hook in an apparatus stabilized at a temperature of 40±2°C, left for 30 min, attached with a load (300 g) and left until natural falling occurs. The retention time then was measured. The experiment was performed at n=3, and the total 3 points were averaged. The results thereof are shown in Table 5.

### [Constant load peeling test]

A sample is cut in 12 mm, length 50 mm and one end (about 5 mm) is peeled off and reinforced with an auxiliary sheet. The test piece is pressed against a bakelite (phenol resin) plate by one reciprocation of a roller (weight 850 g). After 30 min, the test piece is peeled off from the plate until the length of the adhered part of the test piece is 30 mm. The test piece was placed horizontally on a test table in a water bath at 40±2°C, a 30 g load was set on the auxiliary sheet, and the time necessary for the test piece to fall from the test plate was measured and the peeling rate of the preparation in the presence of water was determined. When the test piece does not fall after lapse of 30 min, the peeling length at the time point of 30 min is measured by a ruler. The experiment was performed at n=3, and the total 3 points were averaged. The results thereof are shown in Table 5.

**[Table 5]**

| | compound A (parts) | acrylic polymer A (parts) | IPM (parts) | sesame oil (parts) | lactic acid (parts) | BHT (parts) | magnesium alumino-metasilicate (parts) | PVP (parts) | EOP (parts) | cross-linking agent (parts) | thickness (µm) of adhesive layer | adhesive force (N/24 mm) | holding power (min) | constant load (mm/min) |
|---|---|---|---|---|---|---|---|---|---|---|---|---|---|---|
| Ex. 1B | 6.46 | 55.94 | 17.77 | 14.54 | 3 | 0.97 | 1.26 | - | - | 0.06 | 120 | 2.401 | 2.2 | 0.05 |
| Ex. 2B | 6.46 | 55.94 | 17.77 | 14.54 | 3 | 0.97 | 1.26 | - | - | 0.06 | 60 | 1.986 | 3.8 | 0.36 |
| Ex. 3B | 6.46 | 56.20 | 17.76 | 14.53 | 3 | 0.97 | 1.0 | - | - | 0.08 | 60 | 1.830 | 9.6 | 0.68 |
| Ex. 4B | 6.46 | 54.28 | 17.77 | 14.54 | 3 | 0.97 | 2.9 | - | - | 0.08 | 60 | 1.852 | 8.9 | 0.59 |
| Ex. 5B | 6.59 | 56.37 | 18.13 | 14.83 | 1 | 0.99 | 2.0 | - | - | 0.09 | 60 | 1.902 | 22.4 | 0.21 |
| Ex. 6B | 6.53 | 55.79 | 17.94 | 14.68 | 2 | 0.98 | 2.0 | - | - | 0.08 | 60 | 1.784 | 17.6 | 0.41 |
| Ex. 7B | 6.47 | 55.27 | 17.77 | 14.54 | 3 | 0.97 | 1.9 | - | - | 0.08 | 60 | 1.699 | 10.8 | 0.47 |
| Ex. 8B | 6.47 | 53.34 | 32.31 | - | 3 | - | - | 4.85 | - | 0.03 | 60 | 1.640 | 1.4 | 0.56 |
| Ex. 9B | 6.47 | 55.27 | 32.32 | - | 3 | 0.97 | - | - | 1.94 | 0.03 | 60 | 1.731 | 2.9 | 0.53 |
| Ex. 10B | 6.47 | 55.27 | 32.32 | - | 3 | 0.97 | 1.94 | - | - | 0.03 | 60 | 1.780 | 3.3 | 0.05 |
| Ex. 11B | 6.46 | 56.00 | 17.77 | 14.54 | 3 | 0.97 | 1.26 | - | - | - | 120 | 2.494 | 1.1 | 0.10 |
| Com. Ex. 1B | 6.46 | 57.18 | 17.76 | 14.54 | 3 | 0.97 | - | - | - | 0.09 | 60 | 1.986 | 11.5 | 1.33 |
| Com. Ex. 2B | 6.47 | 58.17 | 32.31 | - | 3 | - | - | - | - | 0.05 | 60 | 1.484 | 3.2 | 2.95 |
| Com. Ex. 3B | 6.47 | 57.21 | 32.32 | - | 3 | 0.97 | - | - | - | 0.03 | 60 | 1.762 | 0.9 | 2.08 |

In Examples 1B - 7B, the amounts of acrylic polymer A, IPM, and the crosslinking agent are almost the same. In all of Examples 1B - 7B, the peeling rate in the presence of water was late in the constant load peeling test as compared to Comparative Example 1B, and it was demonstrated that a patch preparation containing magnesium aluminometasilicate as in Examples 1B - 7B is superior in the adhesiveness in the presence of water.

As shown in Examples 5B - 7B, in a patch preparation wherein the amount of lactic acid is changed relative to magnesium aluminometasilicate, it was confirmed that 0.6 - 2.0 parts by weight of magnesium aluminometasilicate per 1 part by weight of lactic acid is superior in the adhesiveness to the skin in the presence of water. However, it was suggested that the holding power became high and the adhesive layer was rather hard when the amount of magnesium alumino metasilicate was high relative to the amount of lactic acid.

In the constant load peeling test in Examples 8B, 9B and 10B, the detaching speed in the presence of water was practically sufficiently late, which shows good adhesiveness in the presence of water. As compared to the amount of magnesium aluminometasilicate in Example 10B, in Example 8B containing a comparatively large amount of polyvinylpyrrolidone, the adhesive force was almost the same as in Example 10B, but the peeling rate in the presence of water was somewhat fast in the constant load peeling test, and the same tendency was seen in Example 9B containing an aminoalkyl methacrylate copolymer in the same amount as magnesium aluminometasilicate in Example 10B.

Since the results of the constant load peeling test were good and the peeling rate in the presence of water was low even in Example 11B wherein the adhesive layer was not crosslinked, it was confirmed that the adhesiveness to the skin in the presence of water was superior.

### [Example 12B] - [Example 15B]

In the same manner as in Example 1B except that the compositions and the thickness of the adhesive layer shown in the following Table 6 were employed, the patch preparations of [Example 12B] - [Example 15B] were obtained.

The adhesive force measurement test, holding power measurement test, and constant load peeling test of the patch preparations of [Example 12B] - [Example 15B] were performed according to the methods similar to the aforementioned methods. The results thereof are shown in Table 6.

The calculation of the production rate of the analogues of the patch preparations of [Example 12B] - [Example 15B] and the evaluation of coloration thereof were performed according to the methods similar to the methods of the Examples in the first aspect of the present invention. The results thereof are shown in Table 7.

**[Table 6]**

| | compound A (parts) | acrylic polymer A (parts) | IPM (parts) | sesame oil (parts) | lactic acid (parts) | BHT (parts) | magnesium alumino-metasilicate (parts) | crosslinking agent (parts) | thickness of adhesive layer (µm) | adhesive force (N/24 mm) | holding power (min) | constant load (mm/min) |
|---|---|---|---|---|---|---|---|---|---|---|---|---|
| Ex. 12B | 6.5 | 58.1 | 17.8 | 14.5 | 1.5 | 0.3 | 1.3 | - | 80 | 2.745 | 3.4 | 0.09 |
| Ex. 13B | 6.5 | 57.4 | 17.8 | 14.5 | 1.5 | 1 | 1.3 | - | 80 | 2.675 | 2.9 | 0.09 |
| Ex. 14B | 6.5 | 56.4 | 17.8 | 14.5 | 1.5 | 2 | 1.3 | - | 80 | 2.785 | 2.5 | 0.05 |
| Ex. 15B | 6.5 | 53.4 | 17.8 | 14.5 | 1.5 | 5 | 1.3 | - | 80 | 2.646 | 1.9 | 0.05 |

**[Table 7]**

| | rate of total analogue (area %) | color |
|---|---|---|
| Ex. 12B | 0.67 | none |
| Ex. 13B | 0.64 | none |
| Ex. 14B | 0.60 | none |
| Ex. 15B | 0.47 | none |

From the results of Table 6, it was demonstrated that a patch preparation containing magnesium aluminometasilicate was superior in the adhesiveness in the presence of water.

As shown in Table 7, moreover, in a patch preparation wherein the content of BHT in the adhesive layer is changed, it was confirmed that an amount of BHT of 0.3 - 5 wt% in 100 wt% of the adhesive layer showed remarkable suppressive effects on the production of the total analogue of compound A and coloration of the preparation, and stabilized the compound A-containing patch preparation.

### Industrial Applicability

According to the patch preparation (I) of the first aspect of the present invention, an adhesive preparation of blonanserin, which shows reduced irritation and superior preservation stability, can be provided by adding lactic acid, sesame oil and particular additives.

On the other hand, the patch preparation (II) of the present invention can suppress a decrease in the adhesiveness of the patch preparation containing lactic acid in the presence of water, while maintaining a permeation promoting effect afforded by the lactic acid, and can suppress detachment from the skin and the like, by adding at least one kind of filler selected from the group consisting of magnesium aluminometasilicate, polyvinylpyrrolidone and an aminoalkyl methacrylate copolymer to the adhesive layer. Therefore, the second aspect of the present invention can provide a patch preparation superior in both the skin permeability of blonanserin or a physiologically acceptable acid addition salt thereof, and adhesiveness in the presence of water.

## Claims

1. A patch preparation comprising a support and an adhesive layer formed on one surface of the support,
wherein the adhesive layer comprises
(i) 2-(4-ethyl-1-piperazinyl)-4-(4-fluorophenyl)-5,6,7,8,9,10-hexahydrocycloocta[b]pyridine or a physiologically acceptable acid addition salt thereof,
(ii) an acrylic polymer,
(iii) lactic acid,
(iv) sesame oil, and
(v-1) one or more kinds of additives selected from the group consisting of 2-mercaptobenzimidazole, 2,6-di-tert-butyl-4-methylphenol and propyl gallate, and/or
(v-2) a filler comprising one or more kinds selected from the group consisting of magnesium aluminometasilicate, polyvinylpyrrolidone and an aminoalkyl methacrylate copolymer,
and wherein the adhesive layer further comprises (vi) an organic liquid component which is a fatty acid ester formed of a fatty acid having a carbon number of 8 to 18 and a monohydric alcohol having a carbon number of 1 to 18.

2. The patch preparation according to claim 1, wherein the content of the lactic acid in 100 wt% of the adhesive layer is 0.1 - 10 wt%.

3. The patch preparation according to claim 1 or 2, wherein the content of the sesame oil in 100 wt% of the adhesive layer is 3 - 20 wt%.

4. The patch preparation according to any one of claims 1 to 3, wherein the content of the additive in 100 wt% of the adhesive layer is 0.3 - 10 wt%.

5. The patch preparation according to any one of claims 1 to 4, wherein the organic liquid component comprises isopropyl myristate.

6. The patch preparation according to any one of claims 1 to 5, wherein the total content of the organic liquid component and sesame oil in the adhesive layer is 5 - 60 wt%.

7. A patch preparation comprising a support and an adhesive layer formed on one surface of the support, wherein the adhesive layer comprises
(i) 2-(4-ethyl-1-piperazinyl)-4-(4-fluorophenyl)-5,6,7,8,9,10-hexahydrocycloocta[b]pyridine or a physiologically acceptable acid addition salt thereof,
(ii) an acrylic polymer,
(iii) lactic acid, and
(v-2) a filler comprising one or more kinds selected from the group consisting of magnesium aluminometasilicate, polyvinylpyrrolidone and an aminoalkyl methacrylate copolymer,
and wherein the adhesive layer further comprises (vi) an organic liquid component which is a fatty acid ester formed of a fatty acid having a carbon number of 8 to 18 and a monohydric alcohol having a carbon number of 1 to 18.

8. The patch preparation according to claim 7, wherein the filler comprises magnesium aluminometasilicate,
and optionally wherein the magnesium aluminometasilicate is an amorphous composite oxide wherein aluminum, magnesium and a silicon atom are three-dimensionally polymerized via an oxygen atom.

9. The patch preparation according to any one of claims 7 and 8, wherein the adhesive layer is crosslinked.

10. The patch preparation according to any one of claims 7 to 9, wherein the content of the lactic acid in the adhesive layer is 0.1 - 10 wt% in 100 wt% of the adhesive layer.

11. The patch preparation according to any one of claims 7 to 10, wherein the content of the filler in the adhesive layer is 0.1 - 40 wt% in 100 wt% of the adhesive layer.

12. The patch preparation according to any one of claims 7 to 11, wherein the organic liquid component comprises isopropyl myristate.

13. The patch preparation according to any one of claims 7 to 12, wherein the adhesive layer comprises (iv) sesame oil.

14. The patch preparation according to any one of claims 7 to 13, wherein the adhesive layer further comprises one or more kinds of additives selected from (v-1) 2-mercaptobenzimidazole, 2,6-di-tert-butyl-4-methylphenol and propyl gallate,
and optionally wherein the content of the additive in the adhesive layer is 0.3 - 10 wt% in 100 wt% of the adhesive layer.

## Patentansprüche

1. Pflasterpräparat umfassend einen Träger und eine Klebstoffschicht, die auf einer Oberfläche des Trägers gebildet ist,
wobei die Klebstoffschicht umfasst
(i) 2-(4-Ethyl-1-piperazinyl)-4-(4-fluorphenyl)-5,6,7,8,9,10-hexahydrocycloocta[b]pyridin oder ein physiologisch verträgliches Säureadditionssalz davon,
(ii) ein Acrylpolymer,
(iii) Milchsäure,
(iv) Sesamöl, und
(v-1) eine oder mehrere Arten von Additiven, ausgewählt aus der Gruppe bestehend aus 2-Mercaptobenzimidazol, 2,6-Di-tert-butyl-4-methylphenol und Propylgallat, und/oder
(v-2) einen Füllstoff umfassend eine oder mehrere Arten, ausgewählt aus der Gruppe bestehend aus Magnesiumaluminometasilicat, Polyvinylpyrrolidon und einem Aminoalkylmethacrylat-Copolymer,
und wobei die Klebstoffschicht außerdem (vi) eine organische flüssige Komponente umfasst, welche ein Fettsäureester ist, der aus einer Fettsäure mit einer Kohlenstoffzahl von 8 bis 18 und einem einwertigen Alkohol mit einer Kohlenstoffzahl von 1 bis 18 gebildet ist.

2. Pflasterpräparat gemäß Anspruch 1, wobei der Gehalt der Milchsäure in 100 Gew.-% der Klebstoffschicht 0,1 - 10 Gew.-% beträgt.

3. Pflasterpräparat gemäß Anspruch 1 oder 2, wobei der Gehalt des Sesamöls in 100 Gew.-% der Klebstoffschicht 3 - 20 Gew.-% beträgt.

4. Pflasterpräparat gemäß einem der Ansprüche 1 bis 3, wobei der Gehalt des Additivs in 100 Gew.-% der Klebstoffschicht 0,3 - 10 Gew.-% beträgt.

5. Pflasterpräparat gemäß einem der Ansprüche 1 bis 4, wobei die organische flüssige Komponente Isopropylmyristat umfasst.

6. Pflasterpräparat gemäß einem der Ansprüche 1 bis 5, wobei der Gesamtgehalt der organischen flüssigen Komponente und des Sesamöls in der Klebstoffschicht 5 - 60 Gew.-% beträgt.

7. Pflasterpräparat umfassend einen Träger und eine Klebstoffschicht, die auf einer Oberfläche des Trägers gebildet ist, wobei die Klebstoffschicht umfasst
(i) 2-(4-Ethyl-1-piperazinyl)-4-(4-fluorphenyl)-5,6,7,8,9,10-hexahydrocycloocta[b]pyridin oder ein physiologisch verträgliches Säureadditionssalz davon,
(ii) ein Acrylpolymer,
(iii) Milchsäure, und
(v-2) einen Füllstoff umfassend eine oder mehrere Arten, ausgewählt aus der Gruppe bestehend aus Magnesiumaluminometasilicat, Polyvinylpyrrolidon und einem Aminoalkylmethacrylat-Copolymer,
und wobei die Klebstoffschicht außerdem (vi) eine organische flüssige Komponente umfasst, welche ein Fettsäureester ist, der aus einer Fettsäure mit einer Kohlenstoffzahl von 8 bis 18 und einem einwertigen Alkohol mit einer Kohlenstoffzahl von 1 bis 18 gebildet ist.

8. Pflasterpräparat gemäß Anspruch 7, wobei der Füllstoff Magnesiumaluminometasilicat umfasst,
und wobei gegebenenfalls das Magnesiumaluminometasilicat ein amorphes Verbundoxid ist, in dem Aluminium, Magnesium und ein Siliciumatom über ein Sauerstoffatom dreidimensional polymerisiert sind.

9. Pflasterpräparat gemäß einem der Ansprüche 7 und 8, wobei die Klebstoffschicht vernetzt ist.

10. Pflasterpräparat gemäß einem der Ansprüche 7 bis 9, wobei der Gehalt der Milchsäure in der Klebstoffschicht 0,1 - 10 Gew.-% in 100 Gew.-% der Klebstoffschicht beträgt.

11. Pflasterpräparat gemäß einem der Ansprüche 7 bis 10, wobei der Gehalt des Füllstoffs in der Klebstoffschicht 0,1 - 40 Gew.-% in 100 Gew.-% der Klebstoffschicht beträgt.

12. Pflasterpräparat gemäß einem der Ansprüche 7 bis 11, wobei die organische flüssige Komponente Isopropylmyristat umfasst.

13. Pflasterpräparat gemäß einem der Ansprüche 7 bis 12, wobei die Klebstoffschicht (iv) Sesamöl umfasst.

14. Pflasterpräparat gemäß einem der Ansprüche 7 bis 13, wobei die Klebstoffschicht außerdem eine oder mehrere Arten von Additiven, ausgewählt aus (v-1) 2-Mercaptobenzimidazol, 2,6-Di-tert-butyl-4-methylphenol und Propylgallat, umfasst, und wobei gegebenenfalls der Gehalt des Additivs in der Klebstoffschicht 0,3 - 10 Gew.-% in 100 Gew.-% der Klebstoffschicht beträgt.

## Revendications

1. Préparation de patch comprenant un support et une couche adhésive formée sur une surface du support,
dans laquelle la couche adhésive comprend
(i) de la 2-(4-éthyl-1-pipérazinyl)-4-(4-fluoro-phényl)-5,6,7,8,9,10-hexahydrocyclo-octa[b]pyridine ou un sel d'addition d'acide physiologiquement acceptable de celle-ci,
(ii) un polymère acrylique,
(iii) de l'acide lactique,
(iv) de l'huile de sésame, et
(v-1) un ou plusieurs types d'additifs choisis dans le groupe se composant du 2-mercaptobenzimidazole, du 2,6-di-tert-butyl-4-méthylphénol et du gallate de propyle, et/ou
(v-2) une charge comprenant un ou plusieurs types choisis dans le groupe se composant de l'aluminométasilicate de magnésium, de la polyvinylpyrrolidone et d'un copolymère d'aminoalkyl-méthacrylate
et dans laquelle la couche adhésive comprend en outre (vi) un composant liquide organique qui est un ester d'acide gras formé d'un acide gras ayant un nombre de carbones de 8 à 18 et un alcool monohydrique ayant un nombre de carbones de 1 à 18.

2. Préparation de patch selon la revendication 1, dans laquelle la teneur en acide lactique dans 100 % en poids de la couche adhésive est de 0,1 à 10 % en poids.

3. Préparation de patch selon la revendication 1 ou 2, dans laquelle la teneur en huile de sésame dans 100 % en poids de la couche adhésive est de 3 à 20 % en poids.

4. Préparation de patch selon l'une quelconque des revendications 1 à 3, dans laquelle la teneur en additif dans 100 % en poids de la couche adhésive est de 0,3 à 10 % en poids.

5. Préparation de patch selon l'une quelconque des revendications 1 à 4, dans laquelle le composant liquide organique comprend le myristate d'isopropyle.

6. Préparation de patch selon l'une quelconque des revendications 1 à 5, dans laquelle la teneur totale en composant liquide organique et en huile de sésame dans la couche adhésive est de 5 à 60 % en poids.

7. Préparation de patch comprenant un support et une couche adhésive formée sur une surface du support, dans laquelle la couche adhésive comprend
(i) de la 2-(4-éthyl-1-pipérazinyl)-4-(4-fluoro-phényl)-5,6,7,8,9,10-hexahydrocyclo-octa[b]pyridine ou un sel d'addition d'acide physiologiquement acceptable de celle-ci,
(ii) un polymère acrylique,
(iii) de l'acide lactique, et
(v-2) une charge comprenant un ou plusieurs types choisis dans le groupe se composant de l'aluminométasilicate de magnésium, de la polyvinylpyrrolidone et d'un copolymère d'aminoalkyl-méthacrylate,
et dans laquelle la couche adhésive comprend en outre (vi) un composant liquide organique qui est un ester d'acide gras formé d'un acide gras ayant un nombre de carbones de 8 à 18 et un alcool monohydrique ayant un nombre de carbones de 1 à 18.

8. Préparation de patch selon la revendication 7, dans laquelle la charge comprend de l'aluminométasilicate de magnésium,
et éventuellement dans laquelle l'aluminométasilicate de magnésium est un oxyde composite amorphe où l'aluminium, le magnésium et un atome de silicium sont polymérisés tridimensionnellement par le biais d'un atome d'oxygène.

9. Préparation de patch selon l'une quelconque des revendications 7 et 8, dans laquelle la couche adhésive est réticulée.

10. Préparation de patch selon l'une quelconque des revendications 7 à 9, dans laquelle la teneur en acide lactique dans la couche adhésive est de 0,1 à 10 % en poids dans 100 % en poids de la couche adhésive.

11. Préparation de patch selon l'une quelconque des revendications 7 à 10, dans laquelle la teneur en charge dans la couche adhésive est de 0,1 à 40 % en poids dans 100 % en poids de la couche adhésive.

12. Préparation de patch selon l'une quelconque des revendications 7 à 11, dans laquelle le composant liquide organique comprend du myristate d'isopropyle.

13. Préparation de patch selon l'une quelconque des revendications 7 à 12, dans laquelle la couche adhésive comprend (iv) de l'huile de sésame.

14. Préparation de patch selon l'une quelconque des revendications 7 à 13, dans laquelle la couche adhésive comprend en outre un ou plusieurs types d'additifs choisis parmi (v-1) le 2-mercaptobenzimidazole, le 2,6-di-tert-butyl-4-méthylphénol et le gallate de propyle,
et éventuellement dans laquelle la teneur en additif dans la couche adhésive est de 0,3 à 10 % en poids dans 100 % en poids de la couche adhésive.
